(19) Europäisches Patentamt
European Patent Office
Office européen des brevets

(11) EP 2 985 347 B1

(12) **EUROPEAN PATENT SPECIFICATION**

(45) Date of publication and mention of the grant of the patent:
**06.01.2021 Bulletin 2021/01**

(21) Application number: **14782975.8**

(22) Date of filing: **11.04.2014**

(51) Int Cl.:
***C12N 15/62** (2006.01)* ***C12N 15/65** (2006.01)*
***C07K 2/00** (2006.01)* ***G01N 21/64** (2006.01)*

(86) International application number:
**PCT/CN2014/075216**

(87) International publication number:
**WO 2014/166429 (16.10.2014 Gazette 2014/42)**

(54) **METHOD FOR DETECTING PROTEIN STABILITY AND USES THEREOF**

VERFAHREN ZUM NACHWEIS EINER PROTEINSTABILITÄT UND VERWENDUNGEN DAVON

PROCÉDÉ DE DÉTECTION DE LA STABILITÉ DE PROTÉINES ET SES UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2013 CN 201310128492**

(43) Date of publication of application:
**17.02.2016 Bulletin 2016/07**

(73) Proprietor: **Center for Excellence in Molecular Cell Science,
Chinese Academy of Sciences
Xuhui District
Shanghai
200031 (CN)**

(72) Inventors:
- **HU, Ronggui
Shanghai 200031 (CN)**
- **YU, Tao
Shanghai 200031 (CN)**
- **TAO, Yonghui
Shanghai 200031 (CN)**

(74) Representative: **Mewburn Ellis LLP
Aurora Building
Counterslip
Bristol BS1 6BX (GB)**

(56) References cited:
**WO-A2-2004/020458 CN-A- 1 396 265**

- **SUSANA RODRIGUEZ ET AL: "Targeted Chemical-Genetic Regulation of Protein Stability InVivo", CHEMISTRY AND BIOLOGY, CURRENT BIOLOGY, LONDON, GB, vol. 19, no. 3, 28 December 2011 (2011-12-28), pages 391-398, XP028475185, ISSN: 1074-5521, DOI: 10.1016/J.CHEMBIOL.2011.12.022 [retrieved on 2012-01-27]**
- **TAO HU ET AL: "Generation of a stable mammalian cell line for simultaneous expression of multiple genes by using 2A peptide-based lentiviral vector", BIOTECHNOLOGY LETTERS, SPRINGER NETHERLANDS, DORDRECHT, vol. 31, no. 3, 26 November 2008 (2008-11-26), pages 353-359, XP019670263, ISSN: 1573-6776**
- **H.-C. S. YEN ET AL: "Global Protein Stability Profiling in Mammalian Cells", SCIENCE, vol. 322, no. 5903, 7 November 2008 (2008-11-07), pages 918-923, XP055295316, US ISSN: 0036-8075, DOI: 10.1126/science.1160489**
- **JONATHAN D. VERRIER ET AL: "Bicistronic Lentiviruses Containing a Viral 2A Cleavage Sequence Reliably Co-Express Two Proteins and Restore Vision to an Animal Model of LCA1", PLOS ONE, vol. 6, no. 5, 27 May 2011 (2011-05-27), page e20553, XP055295342, DOI: 10.1371/journal.pone.0020553**
- **XIAOLU L ANG ET AL: "A road map of cellular protein homeostasis", NATURE CHEMICAL BIOLOGY, vol. 5, no. 1, 1 January 2009 (2009-01-01) , pages 9-11, XP055295335, GB ISSN: 1552-4450, DOI: 10.1038/nchembio0109-9**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- TAO YU ET AL: "Profiling human protein degradome delineates cellular responses to proteasomal inhibition and reveals a feedback mechanism in regulating proteasome homeostasis", CELL RESEARCH - XIBAO YANJIU, vol. 24, no. 10, 16 September 2014 (2014-09-16), pages 1214-1230, XP055295186, GB, CN ISSN: 1001-0602, DOI: 10.1038/cr.2014.122
- GANESAN, S. ET AL.: 'A dark yellow fluorescent protein (YFP)-based Resonance Energy-Accepting Chromoprotein (REACh) for Forster resonance energy transfer with GFP.' PNAS. vol. 103, no. 11, 14 March 2006, pages 4089 - 4094, XP055286606
- VARSHAVSKY ALEXANDER: "Ubiquitin fusion technique and related methods", METHODS IN ENZYMO, ACADEMIC PRESS, US, vol. 399, 1 January 2005 (2005-01-01), pages 777-799, XP008164532, ISSN: 0076-6879

## Description

### Technical field

The present invention relates to the field of detection of proteins, and in particular, to a method for detecting protein stability and uses thereof.

### Background

Protein dynamics in protein level are essential features and prerequisites for a cell to maintain viability and perform life activities. At any time, the level of a specific protein in a cell depends on the dynamic equilibrium between synthesis and degradation of the protein. Proteins are mainly synthesized on ribosome, and at present, processes mechanisms for the synthesis and regulation of a protein are well understood.

Research on protein degradation in human began in 1980's, and in recent years, has become a very important area on modern biology, because basic life processes of a cell, such as cell cycle, are not possible, unless all of the proteins are timely degraded. Numerous evidences showed that many damaged or misfolded proteins must be degraded; otherwise normal cell function will be affected, resulting in diseases such as cancer, neurodegenerative diseases (C. Raiborg and H. Stenmark, 2009; Christian Hirsch et, 2009; Steven Bergink and Stefan Jentsch, 2009).

Abnormal protein degradation pathways are involved in many pathological processes (Salvatore Oddo, 2008; Jeffrey H Kordower, 2008; Ji anjun Wang, 2008), therefore, many tumor cells and viruses also take advantage of the ubiquitin-proteasome system to disrupt protein degradation, so as to achieve the distribution of tumor cells or their invasion into host cells (Daniela Hoeller and Ivan Dikic, 2009). Many drugs which can affect protein degradation pathways have been or are being developed. Existing research results and data from clinical trials have demonstrated that these drugs have high potential medicinal value for many currently incurable diseases (Matthew D Petroski, 2008; Grzegorz Nalepa, 2006).

Therefore, studies on the change in protein stability will provide important insights on the forecast, incidence, etiology of the protein-involved diseases, and the screening of therapeutic agents. Particularly, the detection of abnormal stability of a protein is very important for the study of pathogenesis. At present, the stability of a protein is detected through cycloheximide chase assay. However, this method is labor-consuming, and the changes in protein stability within the scope of the proteomics can not be studied. And such assay is not suitable for high-throughput drug screening due to its complexity.

By ProteinChip technology, the stabilities of multiple proteins can be detected in one time. However, the throughput of the technology is limited due to the availability of antibodies, thereby greatly limiting the practicability of the technology. Changes in protein stability within the scope of proteomics can be detected by mass spectrometry in large-scale samples. However, mass spectrometry has limited capability of identification, and changes in stability of low-abundance protein can not be detected.

In the field of cell biology and molecular biology, the fluorescent proteins are widely used. Fluorescent proteins are often used as reporter genes to identify the cellular localization of a target protein, and can be used in combination with FACS technology for relatively quantitating intracellular abundance of a target protein.

Therefore, there is an urgent need in the art to develop a novel detection method, which can be widely used for genome-wide detection of the stability of a protein, and readily applied to high-throughput screen of small compounds.

### Summary of invention

The invention is as defined in the appended claims.

The aim of the present disclosure is to provide a fusion protein for detecting protein stability and a genetic construct encoding the fusion protein, a vector comprising said genetic construct, a cell comprising said genetic construct or vector, and a library consisting of said cell, and a method for detecting protein stability and uses thereof. The method of the present disclosure for detecting protein stability is of high sensitivity and specificity, and easy to operate.

In the first aspect, the present disclosure provides a genetic construct as follows:
5'-A + B + C + D + E-3', wherein,

A represents a promoter;
B represents a coding sequence for a fusion protein consisting of a target protein and a first marker protein;
C represents a coding sequence for a linker peptide;
D represents a coding sequence for a second marker protein; and
E represents a terminator;
wherein B and D can be interchanged.

In a preferred embodiment, the promoter is a CMV promoter.

In another preferred embodiment, B, from 5' to 3', comprises a coding sequence for the target protein and a coding sequence for the first marker protein.

In another preferred embodiment, the linker peptide is selected from: ubiquitin, truncated ubiquitin, ubiquitin mutants and ubiquitin-like proteins, 2A peptides and the like; preferably, ubiquitin; most preferably, ubiquitin with saturation K(R) mutation.

In a preferred embodiment, the first marker protein and the second marker protein are fluorescent proteins.

In a preferred embodiment, the fluorescent protein is selected from EGFP or RFP; and preferably, the first marker protein is EGFP, and the second marker protein is RFP.

In a preferred embodiment, EGFP is mEGFP, and RFP is mRFP.

In a preferred embodiment, one or more tags, such as Flag or myc, are fused at C-terminal or N-terminal of the fluorescent protein.

In another preferred embodiment, the coding sequence of the target protein comprises all of the genes in human genomic library human ORFeome V5.1.

In a second aspect, the present disclosure provides a polypeptide with a structure of the following formula:

$$B1 + C1 + D1,$$

wherein,

B1 represents a fusion protein consisting of a target protein and a first marker protein;
C1 represents a linker peptide; and
D1 represents a second marker protein;
wherein B1 and D1 can be interchanged.

In a preferred embodiment, each part in the polypeptide successively is the target protein, the first marker protein, linker peptide and the second marker protein, or the second marker protein, the linker peptide, the target protein and the first marker protein.

In a preferred embodiment, the polypeptide is encoded by genetic construct of claim 1.

In another preferred embodiment, the linker peptide is selected from: ubiquitin, ubiquitin mutants and ubiquitin like proteins, 2A peptides and the like; preferably, ubiquitin; most preferably, ubiquitin with saturation K(R) mutation.

In another preferred embodiment, the first marker protein and the second marker protein are fluorescent proteins.

In a preferred embodiment, the fluorescent protein is selected from EGFP or RFP; and preferably, the first marker protein is EGFP, and the second marker protein is RFP.

In another preferred embodiment, EGFP is mEGFP, and RFP is mRFP.

In another preferred embodiment, one or more tags, such as Flag or myc, are fused at C-terminal or N-terminal of the fluorescent protein.

In another preferred embodiment, the target protein comprises all of the proteins encoded by open reading frames in human genomic library human ORFeome V5.1.

In the third aspect, the present disclosure provides a vector comprising the genetic construct according to the first aspect of the present disclosure.

In a preferred embodiment, the vector can be a plasmid, such as a retroviral plasmid, a lentivirus plasmid, which can be integrated into cell genome of a recipient.

In the fourth aspect, the present disclosure provides a cell comprising the genetic construct according to the first aspect of the present disclosure, or the vector according to the third aspect of the present disclosure.

In the fifth aspect, the present disclosure provides a cell library consisting of the cells of the fourth aspect of the present disclosure.

In a preferred embodiment, said cell is a mammalian cell;

In another preferred embodiment, the mammalian cell is a primate cell or a human cell; and preferably, the mammalian cell is a human cell.

In another preferred embodiment, the mammalian cells include, but are not limited to: 293 cells, 293T cells, 293FT cells, Hela cells, NIH3T3 cells, cancer cells, stem cells, and the like.

In the sixth aspect, the present disclosure provides a method for detecting the stability of one or more target proteins, comprising the following steps:

(1) constructing the cell library according to the fifth aspect of the present disclosure;
(2) culturing the cell library obtained in (1) under the specific conditions and control conditions;

(3) determining the ratio of the first marker protein to the second marker protein in the cell library cultured under the specific conditions and control conditions; and

(4) drawing a conclusion that the stability of the target protein is changed or not, based on the ratio of the first marker protein to the second marker protein in the cell library cultured under the specific conditions and control conditions; or: separating the cell into n sections ($n \geq 2$) through flow cytometry based on the ratio of the first marker protein to the second marker protein, detecting the expression of each target gene in each section by DNA chip technology to calculate the distribution of the target genes in each section, and comparing the distributions of target genes in cell libraries cultured under the specific conditions and control conditions, thereby drawing a conclusion that the stability of the protein encoded by the target gene is changed or not.

In a preferred embodiment, the specific condition can be the addition of a stimulus and disturbance, for example, a chemical stimulus, such as a drug; gene overexpression, knockout or RNA interference; viral infection, stable transfection, plasmid transient transfection, or can be a control condition itself.

In a preferred embodiment, the specific condition refers to the contact with a compound to be tested, stress, radiation or other conditions; and preferably, the specific condition refers to the contact with a compound to be tested.

In a preferred embodiment, the ratio of the first marker protein to the second marker protein, i.e., the ratio of fluorescence intensities of the fluorescent proteins is detected by flow cytometry.

In a preferred embodiment, the number of the sections is 8.

In a preferred embodiment, the target protein comprises all of the proteins encoded by all of ORF in human genomic library human ORFeome V5.1.

It should be understood that in the present disclosure, the technical features specifically mentioned above and below (such as in the Examples) can be combined with each other, thereby constituting a new or preferred technical solution which needs not be individually described.

## DESCRIPTION OF THE DRAWINGS

Figure 1a is a schematic diagram of the construct of a dual-fluorescent plasmid.

Figure 1b is the principle of detecting the relative stability of a protein by using dual-fluorescent plasmid system.

Figure 1c shows that mEGFP without fusion of protein ORF is stable, and forms a stable molecule with mRFP at a molar ratio of 1: 1.

Figure 1d shows that RGS4, the substrate protein of anticancer drugs Bortezomib, was successfully screened using dual-fluorescence plasmid system, and there is no change for negative protein. Western data validation is attached.

Figure 2a is a schematic diagram of genome-wide determination of the stability of a protein by using dual-fluorescence cell library.

Figure 2b is PSI data distribution of genome-wide determination of the relative stability of a protein by using dual-fluorescence cell library.

Figures 2c and d show PSI data of randomly selected genes.

Figure 3a shows the chemical structure of anticancer drug Bortezomib.

Figure 3b shows the overall changes of whole dual-fluorescence cell library upon treatment of anticancer drug Bortezomib.

Figure 3c shows representative data.

Figure 3d is $\Delta$ PSI distribution representing the changes in protein relative stability.

Figures 3e, f, g and h show data validation of chip results using FACS and Western Blot.

Figure 4a shows GO (Biological Pathway) analysis on proteins with dramatic changes in stability using bioinformatics tool DAVID.

Figure 4b shows the detail analysis performed on BP004 subfamily in the results of Figure 4a.

Figure 4c shows the detail analysis performed on BP00179 subfamily in the results of Figure 4a.

Figure 4d shows protein-protein interactions (PPI) analysis performed on response proteins by using bioinformatics tool STRING and visualization tool cytoscape.

Figure 5 shows scatter plots of the control dual-fluorescent cell line.

Figure 6 shows the dual-fluorescent cell library containing human ORFeome V5.1.

Figure 7, using TP53-mEGFPfu-mRFPf as an example, shows the results of western blot of original band of the fusion polypeptide which has just been translated, and two different bands (TP53-mEGFP fusion protein band and mRFP band) formed through cleavage by deubiquitinating enzyme.

Figure 8 shows small compound-protein interactions (CPI) analysis performed on response proteins by using bioinformatics tool STITCH and visualization tool cytoscape.

Figures 9a and b show the results from using BTZ in combination with 17-AAG against CZ-1 or CZ-1 / R cells.
Figures 9c and d show the results from using BTZ in combination with C75 against CZ-1 or CZ-1 / R cells.
Figure 10a shows CI calculation results for the combination of BTZ with C75.
Figure 10b shows CI calculation results for the combination of BTZ with 17-AAG.

## DETAILED DESCRIPTION

Through comprehensive and intensive research, the inventors have unexpectedly found that a dual-fluorescence plasmid system of the present disclosure can be used to achieve an easy and accurate genome-wide detection of the protein stability, thereby significantly improving the practicability of dual fluorescence technology, and such technology can be further applied to high-throughput screening of small compounds affecting the stability of proteins of pathogenic genes and find unknown key genes involved in pathogenesis of diseases.

In the present disclosure, the inventors have designed a dual-fluorescent plasmid system for relative quantification of the protein degradation rate. The key point of the technology is co-expression of a fluorescent reporter protein molecule (mEGFP) which is fused to a specific target protein, and a control fluorescent protein molecule (mRFP), both of which are connected by a specific linker protein, and upon the translation, will be effectively cleaved and separated. The expression level of mRFP serves as an internal reference, and mEGFP is fused to C-terminus of the target protein X, and serve as a measure for intracellular abundance of target protein X. Through mEGFP / mRFP ratio, the inventor can accurately define the stability of the specific protein X in a single cell.

And if any biological event (such as deletion or overexpression of E3 ligase for adjusting the specific protein X) or chemical stimulus (such as drug treatment) selectively alters the stability of the fusion protein X-mEGFP, the abundance of the fusion protein X-mEGFP will be changed, however, the abundance of mRFP won't be affected, thereby changing the mEGFP / mRFP ratio.

Accordingly, the present disclosure can effectively overcome the disadvantages in the prior art and widely applied to the genome-wide determination of the stability of a protein and high-throughput screening of small compounds.

In the present disclosure, a fusion protein consisting of a target protein, a first marker protein, linker peptide and a second marker protein is co-expressed under the same promoter, and a fusion protein of the target protein and the first marker protein and the second protein form respectively upon cutting at the linker peptide, thereby characterizing the relative stability of the target protein by comparison between the first marker protein fused to the target protein and the second marker protein.

For example, in a specific embodiment, according to the present invention, a fluorescent protein is fused to a target gene or a gene library, and the amount of the fusion protein can be characterized based on fluorescent intensity since fluorescent intensity of a fluorescent molecule can be quantitatively determined, so as to obtain parameters regarding protein stability. The simultaneous genome-wide detection of stability of all proteins can be achieved by combining with human ORFeome library genes.

Using this technique, small compounds which can affect the stability of proteins of pathogenic genes can be selected through high-throughput screening. Meanwhile, by combining with human ORFemoe library, genome-wide detection of the stability of a protein can be achieved, and a protein, the stability of which changes between disease state and normal state, can be genome-widely screened, so as to find unknown key genes involved in pathogenesis of diseases for understanding diseases which can not be thoroughly understood yet.

In a preferred embodiment, the dual-fluorescent expression plasmid according to the present invention is constructed as follows:

a control protein ((Red fluorescent protein (RFP)) and a specific target protein fused to a fluorescent reporter protein (enhanced green fluorescent protein (EGFP)) are co-expressed under the same promoter, both of which are connected by ubiquitin, and upon translation, ubiquitin will be effectively cleaved by hundreds of deubiquitinating enzymes in a cell so as to form separated RFP. The expression level of RFP serves as an internal reference, and EGFP is fused to C-terminus of the target protein X and become a measure means for intracellular abundance of target protein X. Through EGFP / RFP ratio, the relative stability of the specific protein X in a single cell can be accurately defined, and cells with different EGFP / RFP ratios can be sorted and detected by flow cytometry. When performing high-throughput screening of drugs, the high-throughput screening can be conducted by linking a target protein into the dual-fluorescent expression vector through efficient homologous recombination technology Gateway reaction, and then constructing a cell line. When it is desired to genome-widely detect abnormal changes in the stability of a protein, all of the genes in human ORFemoe library are recombinantly linked into the dual-fluorescent expression plasmid through homologous recombination technology Gateway reaction, and then cells are infected at ultra-low viral titers (MOI ~0.05), a cell library into which human ORFemoe library is integrated is constructed at a frequency of one-gene per one-cell, and genome-wide detection of the stability of all the proteins can be conducted in one time by using such cell library in combination of FACS and chip technology.

Accordingly, the present disclosure provides a genetic construct with a structure shown in the following formula:

5'-A + B + C + D + E-3', wherein,

A represents a promoter; B represents a coding sequence for a fusion protein consisting of a target protein and a first marker protein; C represents a coding sequence for a linker peptide; D represents a coding sequence for a second marker protein; and E represents a terminator; wherein B and D can be interchanged.

Wherein, the order of the coding sequence for the target protein and the first marker protein can be interchanged.

In light of the teachings of the present disclosure and the prior art, a person skilled in the art will appreciate that, in the present disclosure, a fusion protein consisting of a target protein, a first marker protein, linker peptide and a second marker protein is co-expressed, a fusion protein of the target protein and the first marker protein and the second protein form respectively upon cleaving at the linker peptide, thereby characterizing the relative stability of the target protein by comparison between the first marker protein fused to the target protein and the second marker protein. Therefore, B and D in the above genetic construct can be interchanged without affecting the function or use thereof.

In light of the teachings of the present disclosure and the prior art, a person skilled in the art will further appreciate that the order of the coding sequence for the target protein and the coding sequence for the first marker protein in B can be interchanged without affecting the function or use thereof.

In a preferred embodiment, B, from 5' to 3', comprises the coding sequence for the target protein and the coding sequence for the first marker protein, respectively.

In light of the teachings of the present disclosure and the prior art, a person skilled in the art will appreciate that various of linker peptide can be used in the present disclosure, as long as the linker peptide can be intracellularly cleaved to form two separate marker proteins. For example, in a particular embodiment, the linker peptide is selected from: ubiquitin (Ub), ubiquitin mutants and ubiquitin-like proteins, 2A short peptides and the like; preferably ubiquitin; most preferably, ubiquitin with K(R) mutation. Ubiquitin with K(R) mutation can be used to ensure effective cleavage, while won't become a ubiquitination site, and thus the degradation properties of the target protein will not be affected.

A person skilled in the art will further appreciate that various marker proteins can be used in the present disclosure, as long as the amount of the marker protein can be accurately characterized. For example, in a particular embodiment, the first and the second marker protein are fluorescent proteins; preferably, the fluorescent protein is EGFP or RFP; and more preferably, the first marker protein is EGFP, and the second marker protein is RFP. In a more preferred embodiment, EGFP is EGFP in monomer form (mEGFP), and RFP is RFP in monomer form (mRFP).

In another preferred embodiment, one or more label proteins, such as Flag or myc, are fused at C-terminal or N-terminal of the fluorescent protein.

In another preferred embodiment, the promoter is a CMV promoter.

In another preferred embodiment, the coding sequences of the target proteins are all of the genes in human genomic library.

In the present disclosure, a polypeptide with a structure of the following formula is further provided:

$$B1 + C1 + D1,$$

wherein,

B1 represents a fusion protein consisting of a target protein and a first marker protein;
C1 represents a linker peptide; and
D1 represents a second marker protein;
wherein B1 and D1 can be interchanged.

As said above, a person skilled in the art will appreciate that B1 and D1 in the polypeptide of the present disclosure can be interchanged without affecting the function or use thereof. And the order of the target protein and the first marker protein in the polypeptide of the present disclosure can be interchanged without affecting the function or use thereof.

In a preferred embodiment, the polypeptide is encoded by the genetic construct of the present disclosure.

In a preferred embodiment, the order of each part in the polypeptide is the target protein, the first marker protein, the linker peptide and the second marker protein, or the second marker protein, the linker peptide, the target protein and the first marker protein.

Based on the genetic construct of the present invention, a vector comprising the genetic construct of the present invention, a mammalian cell comprising the genetic construct or the vector, and a mammalian cell library consisting of the mammalian cells are also provided in the present invention.

In a specific embodiment, the vector can be a plasmid, such as a retroviral plasmid, a lentivirus plasmid, which can be integrated into cell genome of a recipient. Said mammalian cell can be a primate cell or a human cell; and preferably, said mammalian cell is a human cell. In a specific embodiment, the mammalian cell includes, but not limited to: 293 cells, and can be other cells, such as 293t, hela, 3t3, cancer cell, stem cell, and the like.

Based on the genetic construct, vector, polypeptide, cell and cell library of the present invention, a method for detecting the change in the stability of a target protein under specific conditions, comprising the following steps:

(1) constructing the cell library according to the present invention;
(2) culturing the obtained cell library under the specific conditions and control conditions;
(3) determining the ratio of the first marker protein to the second marker protein in the cell library cultured under the specific conditions and control conditions; and
(4) drawing a conclusion that the stability of the target protein is changed or not, based on the ratio of the first marker protein to the second marker protein in the cell library cultured under the specific conditions and control conditions.

After the cell library is obtained, the addition of a stimulus and disturbance can be applied, for example, a chemical stimulus, such as a drug; gene overexpression or RNA interference; viral stable transfection, or plasmid instantaneous transfection.

In a preferred embodiment, the specific condition refers to the contact with a compound to be tested, stress, radiation and other conditions; and preferably, the specific condition refers to the contact with a compound to be tested.

In a preferred embodiment, DNA microarrays are used to detect the ratio of the first marker to the second marker protein, for example, to detect the ratio of fluorescence intensity of the first marker to that of the second marker protein.

In a preferred embodiment, the target proteins are all of the proteins in human cell genomic library human ORFeome V5.1.

In the present disclosure, the involved genome-wide gene can be human ORF version 5.1, or higher version or other versions, or can be ORF library versions of other species.

In light of the teachings of the present disclosure and the prior art, a person skilled in the art will appreciate that a variety of marker proteins can be used in the present disclosure, so long as the amount of the marker proteins can be accurately characterized. For example, a variety of fluorescent proteins can be used in the present disclosure, including but not limited to mEGFP and mRFP or a mutant thereof; alternatively, other fluorescent proteins can be used, as long as two fluorescent proteins possess different excitation light wavelength.

Two different fluorescent proteins are used in the polypeptides of the present disclosure. In a preferred embodiment, the fluorescent proteins used in the present disclosure are mEGFP and mRFP.

For example, the fluorescent protein of SEQ ID NO: 1, 2, 3 or 4 can be used in the present disclosure (SEQ ID NO: 1,
atggtgagcaagggcgaggagctgttcaccggggtggtgcccatcctggtcgagctggacggcgacgtaaacggccacaagttcagcgtgtccggcgagggcga
gggcgatgccacctacggcaagctgaccctgaagttcatctgcaccaccggcaagctgcccgtgccctggcccaccctcgtgaccaccctgacctacggcgtgca
gtgcttcagccgctaccccgaccacatgaagcagcacgacttcttcaagtccgccatgcccgaaggctacgtccaggagcgcaccatcttcttcaaggacgacggc
aactacaagacccgcgccgaggtgaagttcgagggcgacaccctggtgaaccgcatcgagctgaagggcatcgacttcaaggaggacggcaacatcctggggc
acaagctggagtacaactacaacagccacaacgtctatatcatggccgacaagcagaagaacggcatcaaggtgaacttcaagatccgccacaacatcgagga
cggcagcgtgcagctcgccgaccactaccagcagaacacccccatcggcgacggccccgtgctgctgcccgacaaccactacctgagcacccagtccgccctg
agcaaagaccccaacgagaagcgcgatcac atggtcctgctggagttcgtgaccgccgccgggatcactctcggcatggacgagctgtacaag; SEQ ID NO:
2, MVSKGEELFTGWPILVELDGDVNGHKFSVSGEGEGDATYGKLTLKFICTTGKLPV PWPTLVTTLTYGVQCFSRYP-
DHMKQH DFFKSAMPEGYVQERTIFFKDDGNYKTRA EVKFEGDTLVNRIELKGIDFKEDGNILGHKLEYNYNSHNVYI-
MADKQKNGIKVNF KIRHNIEDGSVQLADHYQQNTPIGDGPVLLPDNHYLSTQSK(A)LSKDPNEKRDHM VLLE-
FVTAAGITLGMDELYK; SEQ ID NO: 3, atggcctcctccgaggacgtcatcaaggagttcatgcgcttcaaggtgc gcatggagggctccgtgaacg-
gccacgagttcgagatcgagggcgagggcgagggccgcccctacgagggcacccagaccgccaagctgaaggtgaccaagggcggccccctgcccttcgcct
gggacatcctgtcccctcagttccagtacggctccaaggcctacgtgaagcaccccgccgacatccccgactacttgaagctgtccttccccgagggcttcaagtggg
agcgcgtgatgaacttcgaggacggcggcgtggtgaccgtgacccaggactcctccctgcaggacggcgagttcatctacaaggtgaagctgcgc
ggcaccaacttcccctccgacggccccgtaatgcagaagaagaccatgggctgggaggcctccaccgagcggatgtaccccga
ggacggcgccctgaagggcgagatcaagatgaggctgaagctgaaggacggcggccactacgacgccgaggtcaagaccac
ctacatggccaagaagcccgtgcagctgcccggcgcctacaagaccgacatcaagctggacatcacctcccacaacgaggacta
caccatcgtggaacagtacgagcgcgccgagggccgccactccaccggcgcc; SEQ ID NO: 4, MASSEDVIKE
FMRFKVRMEGSVNGHEFEIEGEGEGRPYEGTQTAKLKVTKGGPLPFAWDILSPQF QYGSKAYVKHPADIPDYLKLSF
PEGFKWERVMNFEDGGVVTVTQDSSLQDGEFIY KVKLRGTNFPSDGPVMQKKTMGWEASTERMYPEDGALKGEIK-
MRLKLKDGGHY DAEVKTTYMAKKPVQLPGAYKTDIKLDITSHNEDYTIVEQYERAEGRHSTGA).

The present disclosure includes a fluorescent protein comprising the amino sequence of SEQ ID NO: 2 or 4, and mutants thereof having the same function as SEQ ID NO: 2 or 4, or other fluorescent proteins. Possible fluorescent proteins are listed in Table 1, including but not limited to the following proteins:

Table 1

| Protein (acronym) | Ex/nm | Em/nm | EC/10-3 M-1 cm-1 | QY | quarternary structure | Relative brightness (% EGFP) |
|---|---|---|---|---|---|---|
| Blue fluorescent protein | | | | | | |
| Sirius | 355 | 424 | 15 | 0.24 | monomer* | 11 |
| Azurite | 384 | 448 | 26.2 | 0.55 | monomer* | 43 |
| EBFP | 383 | 445 | 29 | 0.31 | monomer* | 27 |
| EBFP2 | 383 | 448 | 32 | 0.56 | monomer* | 53 |
| Cyan fluorescent protein | | | | | | |
| ECFP | 439 | 476 | 32.5 | 0.4 | monomer* | 39 |
| Cerulean | 433 | 475 | 43 | 0.62 | monomer* | 79 |
| CyPet | 435 | 477 | 35 | 0.51 | monomer* | 53 |
| SCFP | 433 | 474 | 30 | 0.5 | monomer* | 45 |
| Green fluorescent protein | | | | | | |
| EGFP | 488 | 507 | 56 | 0.6 | monomer* | 100 |
| Emerald | 487 | 509 | 57.5 | 0.68 | monomer* | 116 |
| Superfolder avGFP | 485 | 510 | 83.3 | 0.65 | monomer* | 160 |
| T-Sapphire | 399 | 511 | 44 | 0.6 | monomer* | 79 |
| Yellow fluorescent protein | | | | | | |
| EYFP | 514 | 527 | 83.4 | 0.61 | monomer* | 151 |
| Topaz | 514 | 527 | 94.5 | 0.6 | monomer* | 169 |
| Venus | 515 | 528 | 92.2 | 0.57 | monomer* | 156 |
| Citrine | 516 | 529 | 77 | 0.76 | monomer* | 174 |
| YPet | 517 | 530 | 104 | 0.77 | monomer* | 238 |
| SYFP | 515 | 527 | 101 | 0.68 | monomer* | 204 |
| mAmetrine | 406 | 526 | 45 | 0.58 | monomer | 78 |
| Blue fluorescent protein | | | | | | |
| mTagBFP | 399 | 456 | 52 | 0.63 | monomer | 98 |
| Cyan fluorescent protein | | | | | | |
| TagCFP | 458 | 480 | 37 | 0.57 | monomer | 63 |
| AmCyan | 458 | 489 | 44 | 0.24 | tetramer | 31 |
| Midoriishi Cyan | 472 | 495 | 27.3 | 0.9 | dimmer | 73 |
| mTFP1 | 462 | 492 | 64 | 0.85 | monomer | 162 |
| Green fluorescent protein | | | | | | |
| Azami Green | 492 | 505 | 55 | 0.74 | monomer | 121 |
| mWasabi | 493 | 509 | 70 | 0.8 | monomer | 167 |
| ZsGreen | 493 | 505 | 43 | 0.91 | tetramer | 117 |
| TagGFP | 482 | 505 | 58.2 | 0.59 | monomer | 102 |
| TagGFP2 | 483 | 506 | 56.5 | 0.6 | monomer | 105 |
| TurboGFP | 482 | 502 | 70 | 0.53 | dimer | 112 |
| CopGFP | 482 | 502 | 70 | 0.6 | tetramer | 125 |
| AceGFP | 480 | 505 | 50 | 0.55 | monomer | 82 |
| Yellow fluorescent protein | | | | | | |
| TagYFP | 508 | 524 | 64 | 0.6 | monomer | 118 |
| TurboYFP | 525 | 538 | 105 | 0.53 | monomer | 169 |
| ZsYellow | 529 | 539 | 20.2 | 0.42 | tetramer | 25 |
| PhiYFP | 525 | 537 | 130 | 0.4 | dimmer | 158 |

(continued)

| | | | | | | |
|---|---|---|---|---|---|---|
| Orange fluorescent protein | | | | | | |
| Kusabira Orange | 548 | 559 | 51.6 | 0.6 | monomer | 92 |
| Kusabira Orange2 | 551 | 565 | 63.8 | 0.62 | monomer | 118 |
| mOrange | 548 | 562 | 71 | 0.69 | monomer | 146 |
| mOrange2 | 549 | 565 | 58 | 0.6 | monomer | 104 |
| dTomato | 554 | 581 | 69 | 0.69 | dimmer | 142 |
| dTomato-Tandem | 554 | 581 | 138 | 0.69 | pseudo-monomer | 283 |
| DsRed | 558 | 583 | 75 | 0.79 | tetramer | 176 |
| DsRed2 | 563 | 582 | 43.8 | 0.55 | tetramer | 72 |
| DsRed-Express (T1) | 555 | 584 | 38 | 0.51 | tetramer | 58 |
| DsRed-Express2 | 554 | 586 | 35.6 | 0.42 | tetramer | 45 |
| DsRed-Max | 560 | 589 | 48 | 0.41 | tetramer | 59 |
| DsRed-monomer | 556 | 586 | 35 | 0.1 | monomer | 10 |
| TurboRFP | 553 | 574 | 92 | 0.67 | dimmer | 187 |
| TagRFP | 555 | 584 | 100 | 0.48 | monomer | 142 |
| TagRFP-T | 555 | 584 | 81 | 0.41 | monomer | 99 |
| Red fluorescent protein | | | | | | |
| mRuby | 558 | 605 | 112 | 0.35 | monomer | 117 |
| mApple | 568 | 592 | 75 | 0.49 | monomer | 109 |
| mStrawberry | 574 | 596 | 90 | 0.29 | monomer | 78 |
| AsRed2 | 576 | 592 | 56.2 | 0.05 | tetramer | 8 |
| mRFP1 | 584 | 607 | 50 | 0.25 | monomer | 37 |
| JRed | 584 | 610 | 44 | 0.2 | dimer | 26 |
| mCherry | 587 | 610 | 72 | 0.22 | monomer | 47 |
| eqFP611 | 559 | 611 | 78 | 0.45 | tetramer | 106 |
| tdRFP611 | 558 | 609 | 70 | 0.47 | pseudo-monomer | 98 |
| HcRed1 | 588 | 618 | 20 | 0.015 | dimmer | 1 |
| mRaspberry | 598 | 625 | 86 | 0.15 | monomer | 38 |
| Far infrared fluorescent protein | | | | | | |
| tdRFP639 | 589 | 631 | 90.4 | 0.16 | pseudo-monomer | 43 |
| mKate | 588 | 635 | 31.5 | 0.28 | monomer | 26 |
| mKate2 | 588 | 633 | 62.5 | 0.4 | monomer | 74 |
| Katushka | 588 | 635 | 65 | 0.34 | dimer | 67 |
| tdKatushka | 588 | 633 | 132.5 | 0.37 | pseudo-monomer | 146 |
| HcRed-Tandem | 590 | 637 | 160 | 0.04 | pseudo-monomer | 19 |
| mPlum | 590 | 649 | 41 | 0.1 | monomer | 12 |
| AQ143 | 595 | 655 | 90 | 0.04 | tetramer | 11 |
| Note: In the table, the peak excitation wavelength is Ex, the peak emission wavelength is Em, molar extinction coefficient is EC, and quantum yield is QY. Brightness value is calculated from the product of molar extinction coefficient and quantum yield divided by EGFP. | | | | | | |

For example, a fluorescent protein, such as mEGFP may be fused, at N terminal, to ORF of a target protein, that is, the used structure of the fusion protein is: ORF-mEGFP-Ub-mRFP; or, fused, at C terminal, to ORF of a target protein, that is, the used structure of the fusion protein is: mEGFP-ORF-Ub-mRFP; alternatively, for example, mRFP can be fused to ORF of a target protein.

The fluorescent molecule of the present disclosure may have 2 Flag labels fused at C-terminal, or not; alternatively, one or more Flag labels can be fused, or other labels, such as myc and the like, can be used. The label can also be fused at N-terminal of the fluorescent molecule.

In practice, based on the present disclosure in combination with the amino acid composition and structure of fluorescent proteins known in the art, a person skilled in the art can select the type of fluorescent protein molecules to be fused at N-terminal or C-terminal by himself based on the experimental requirement and purpose.

The present disclosure includes the use of ORF fused dual-fluorescent protein to detect the stability of a protein in various libraries. The libraries used in the present disclosure can be commercially-available ORF libraries, or libraries constructed by a person skilled in the art themselves, or human ORF, or ORFs of other species, or a part of function domains in ORF, or a sequence fragment designed by a person skilled in the art himself.

Recombination and amplification of genes in a library are performed in *E. coli.* Competent cells which can absorb DNA can be harvested after exponential growth phase, and treated with $CaCl_2$, the procedure of which is well known in the art. Another method is to use $MgCl_2$. If desired, transformation can be conducted by electroporation.

Once sequences of library genes for constructing a library are obtained, gene library of fused dual-fluorescent protein molecules can be constructed. The construction method can be gene recombination method, for example, Gateway reaction, or other recombination reactions. The method may also be conventional recombinant DNA techniques (Science, 1984; 224: 1431), for example conventional digestion-ligase linking Method.

Upon obtaining a gene library of fused dual-fluorescent protein molecules, dual-fluorescent cell library with integrated plasmid gene can be constructed through viral packaging methods. The plasmid can be lentivirus plasmid or other plasmids into which genome can be integrated, and the cell for constructing the dual-fluorescence cell library can be 293 cell, or can be other cells, such as 293T cell, Hela cell, NIH3T3 cell, cancer cell, stem cell and the like.

The constructed library can be sorted by FASC, and separated into several sections based on mEGFP/mRFP ratio, wherein the number of sections can be 6, 7, 8, 9 or more; alternatively, the cell in some sections with mEGFP/mRFP ratio in the library can be refine-sorted. The number of sections can be 4, 5, 6, 7 or more.

Genome from sorted cells can be extracted respectively through conventional extraction methods for extracting genomic DNA from a cell or tissue and preparing a sample containing genomic DNA. Primers are designed using common segments of plasmid and genomic DNAs as templates. ORFs are amplified through PCR, T7 promoter primer is added to 3' end primer, and tag enzymes used in PCR amplification suitably are enzymes with a strong ability to extend, such as Ex-tag and the like. PCR products are *in vitro* RNA-amplified, amplified products are used in chip hybridization. Chip can be Agilent double standard chip. Results from chips are normalized by using Composite loess normalization in Bioconductor package limma.

The features of the present invention mentioned above, or the features mentioned in the examples, can be optionally combined. Any feature disclosed in the present specification can be used in combination with any other features, and each feature disclosed in the specification can be replaced with alternative feature which can serve an identical, equivalent, or similar purpose. Therefore, the features disclosed herein are only general exemplary examples of the equivalent or similar features, unless specifically indicated otherwise.

Main advantages of the present invention:

1. The practicability of detecting protein stability is greatly improved by the present invention;
2. The method of the present invention is of high detection accuracy, and convenient operation, and time-saving;
3. Changes in protein stability can be detected with high sensitivity and specificity by the method of the present invention;
4. In the method of the present invention, the fluorescent protein molecule is fused to C segment of the target protein, therefore, signal sequence at N terminal of a protein can be effectively exposed, thereby factually simulating dynamic degradation of a protein;
5. In the method of the present invention, ubiquitin Ub is used to link the fluorescent protein molecules, Ub can be high effectively cleaved by large number of intracellular deubiquitinating enzymes, thereby ensuring 1: 1 molar ratio between two fluorescent molecules at the protein translation level;
6. In the method of the present invention, monomeric form of fluorescent molecule is used, which can effectively prevent dynamic change in protein degradation due to the aggregation of fluorescent molecules;
7. The fluorescent molecules used in the present invention exhibits a fast folding rate, thereby ensuring the timely, dynamic tracking of changes in protein stability.

The invention will be further illustrated with reference to the following specific examples. It is to be understood that these examples are only intended to illustrate the invention, but not to limit the scope of the invention. For the experimental methods in the following examples without particular conditions, they are performed under routine conditions, such as conditions described in Sambrook et al., Molecular Cloning: A Laboratory Manual, New York: Cold Spring Harbor Laboratory Press, 1989, or as instructed by the manufacturer. All the percentages or fractions refer to weight percentage and weight fraction, unless stated otherwise.

Unless otherwise defined, all the technical and scientific terms used in the present specification have the meanings as commonly understood by those skilled in the art. In addition, any method and material which are similar or equivalent with the contents disclosed herein can be applied in the present methods. The preferred methods and materials for carrying out the present invention described herein are only given as examples.

**Example**

**Materials and methods**

**CZ-1 cell and CZ-1/R cell**

CZ-1 cell was from multiple myeloma cell line (i.e, BTZ drug-sensitive cell line); this cell line was *in vitro* intermittent-treated by small dose of BTZ, and subcultured for several passages, thereby obtaining BTZ resistance, marked as CZ-1/R (i.e., BTZ-resistant cell line).

CZ-1 cells can be obtained from Hou Jian Laboratory of Shanghai Changzheng Hospital (Hou J, Lin FY, Zhang B, Zhang LZ, Ding SQ, Establishment and biological characteristics of human multiple myeloma cell line CZ-1. Chinese Medical Journal [2004, 117(1):115-119]); CZ-1/R cell lines was constructed by the inventor's Laboratories, and can be obtained from the inventor's laboratory (http://www.sibcb.ac.cn/PI.asp?id=129).

**293 cell**

293 cell was obtained from ATCC (Maryland, USA).

**Construction of plasmid**

Designed dual-fluorescent structures were assembled, and amplified by PCR reaction, and restriction enzyme sites were added, wherein two Flag labels were attached to C-end of mEGFP fragment, two Flag labels were attached to C-end of mRFP fragment, and Ub protein was Ub protein with K(R) saturation mutation, named as $mEGFP_{fu}$-$mRFP_f$. The three fragments were spliced, and inserted into vector pAG426GAL-ccdB-EGFP (Addgene) via Hind III and Xho I restriction sites, wherein EGFP structure was replaced by mEGFP-Ub-mRFP structure, thereby obtaining plasmid pAG426GAL-ccdB-$mEGFP_{fu}$-$mRFP_f$.

Primers for constructing plasmid pAG426GAL-ccdB-$mEGFP_{fu}$-$mRFP_f$ are listed as follows:

HindIII-(a)-mGFPN1(5-3): ACA aagctta atggtgagca agggcgagga(SEQ ID NO: 5)
mGFPC1-FLAG2N1 (3-5): tggacgagct gtacaag ggatctgactacaag(SEQ ID NO: 6)
GWs3(5-3) : gggtctgactacaaggacgatgacgataagggcggagactacaaggacgatgacgataagggctct(SEQ ID NO: 7)
FLAG2C1-hUbN1(5-3): acgatgacgataagggctct atgcagatctttgtgaGg(SEQ ID NO: 8)
GWS5(5-3):

atgcagatctttgtgaGgaccctcactggcaGaaccatcacccttgaggtcgagcccagtgacaccattgagaatgtcaGagccaGaattcaagacaGgg(SEQ ID NO: 9)

GWS6(5-3):

gccaGaattcaagacaGgagggtatcccacctgaccagcagcgtctgatatttgccggcaGacagctggaggatggccgcactctctcagactacaac(SEQ ID NO: 10)

hUbK6RN3-mRFPN1(3-5) :

*cactctctcagactacaacatccagaGagagtccaccctgcacctggtgttgcgcctccgcggtggtatggcctcct ccgaggacgt*(SEQ ID NO: 11)

*GWS8(5-3): atggcctcct ccgaggacgt(SEQ* ID NO: 12)
*GWS9(5-3):* ccgc cactccaccg gcgcc ggAtctgactacaaggacgat(SEQ ID NO: 13)
GWS10(5-3): acaaggacgatgacgataag TAG taactcgagtca(SEQ ID NO: 14)
pAG426GAL-ccdB-mEGFP$_{fu}$-mRFP$_f$ plasmid was digested by spe1 and XhoI restriction endonucleases, and blunted. PCDH-CMV-MCS-EF1a-Puro vector (Addgene) was digested by XbaI, blunted, and then linked by T4 ligase, and sequenced as being correct. Plasmid PCDH-CMV-ccdB-mEGFP$_{fu}$-mRFP$_f$ was obtained.

**Mixing and amplification of human gene libraries**

Human gene libraries were mixed by using Biomek FX automated workstation, and detected for titer. The original libraries were amplified for 100 times, and at 37°C cultured for 18 ~ 24 h. Clones were scraped from the plate, collected and well-mixed. And plasmids were massive-extracted.

**LR reaction and identification of Human gene library**

Human gene library is present in Entry clones containing attL sequence. LR reaction can occur directly between genes in the library and the inventor's target vector PCDH-CMV-ccdB-mEGFP$_{fu}$-mRFP$_f$ (containing attR sequence) (technical details and all of operations can be found in Invitrogen GATEWAY™ Cloning Technology), to obtain human gene libraries with the presence of PCDH-CMV-ORFs-mEGFP$_{fu}$-mRFP$_f$. Clones were scraped, collected and well-mixed. And then plasmids were massive-extracted.

**Construction of dual-fluorescent cell library**

At 37°C and 5% $CO_2$, 293 FT cell line was cultured in DMEM medium containing 10% fetal bovine serum, 10% NEAA and P / S antibiotics in an incubator. 293 FT cells were transfected with pCDH-CMV-ORFs-mEGFP$_{fu}$-mRFP$_f$ library, wherein used helper plasmid was lentiviral expression system (LV100A-1, SBI), and the operation methods can be found in operating specification.

**Construction of dual-fluorescent control cell line**

Plasmids pENTRY-RGS4, pENTRY-TP53, pENTRY-ARC, pENTRY-AXNA1, pENTRY-NFKBIB, and pENTRY-CDC25A were picked from hORFome library, and gateway reaction was conducted with target vector pCDH-CMV-ccdB-mEGFP$_{fu}$-mRFP$_f$, thereby obtaining target plasmids pCDH-CMV-RGS4-mEGFP$_{fu}$-mRFP$_f$, pCDH-CMV-P53-mEGFP$_{fu}$-mRFP$_f$, pCDH-CMV-ARC-mEGFP$_{fu}$-mRFP$_f$, pCDH-CMV-AXNA1-mEGFP$_{fu}$-mRFP$_f$, pCDH-CMV-NFKBIB-mEGFP$_{fu}$-mRFP$_f$, and pCDH-CMV-CDC25A-mEGFP$_{fu}$-mRFP$_f$, respectively. Viruses were packaged by using lentiviral expression system (LV100A-1, SBI). 293 FT cells were infected, and sorted by FACS, thereby obtaining dual-fluorescent overexpressing cell lines pCDH-CMV-RGS4-mEGFP$_{fu}$-mRFP$_f$, pCDH-CMV-TP53-mEGFP$_{fu}$-mRFP$_f$, pCDH-CMV-ARC-mEGFP$_{fu}$-mRFP$_f$, pCDH-CMV-AXNA1-mEGFP$_{fu}$-mRFP$_f$, pCDH-CMV-NFKBIB- mEGFPf$_u$-mRFP$_f$, and pCDH-CMV-CDC25A-mEGFP$_{fu}$-mRFP$_f$, respectively.

Dual-fluorescent overexpressing cell lines pCDH-CMV-RGS4-mEGFP$_{fu}$-mRFP$_f$, pCDH-CMV-TP53-mEGFP$_{fu}$,-mRFP$_f$, pCDH-CMV-ARC-mEGFP$_{fu}$-mRFP$_f$, pCDH-CMV-AXNA1-mEGFP$_{fu}$-mRFP$_f$, pCDH-CMV-NFKBIB-mEGFP$_{fu}$-mRFP$_f$, pCDH-CMV-CDC25A-mEGFP$_{fu}$-mRFP$_f$ were treated by Bortezomib (final concentration of 1 μM) for 6 hrs, with DMSO as the control. Changes in EGFP/RFP ratio were detected by FACS. And then cells were lysed, and changes in the protein abundance were detected by Western assay.

**8-section sort of dual-fluorescent cell library**

Cells in the dual-fluorescence cell library containing Human ORFeome V5.1 genes were digested, blown as single cell, resuspended in a medium, filtered through 40 μm filter, and sorted by FACS AriaII cell sorting system (BD company). When cells were sorted, cell population with high RFP signal was firstly selected, and then the selected cells were sorted into 8 sections based on fluorescence ratio of mEGFP/ mRFP.

**Extraction and microarray hybridization of genomic DNA of cells in sections**

At 55°C, cells in each section were lysed with a lysis buffer for 16 hrs, the composition of which is 10 mM Tris-HCl (8.0), 10 mM EDTA, 0.5% SDS, 0.2 mg / ml proteinase K and 25 μg / ml RNase A. And then 0.2 M sodium chloride was added. Genomic DNA was extracted by phenol-chloroform, purified by chloroform, precipitated by ethanol, and then dissolved in 10 mM Tris-HCl (8.0) and 0.1 mM EDTA buffer. For quantitative amplification of ORF-mEGFP$_{fu}$-mRFP$_f$ structure integrated in the genome, PCR amplification was conducted by using genomic DNA as a template, wherein PCR primers are listed as follows:

Forward primer: CCTGGAGACGCCATCCACGCTG (SEQ ID NO: 15)
Reverse primer:
TAATACGACTCACTATAGGGAGCTCCTCGCCCTTGCTCACCATTAAGCT (SEQ ID NO: 16).

T7 RNA polymerase promoter site was contained in the reverse primer. Amplification was conducted by using EX-Taq enzyme (Takara). And then, using PCR product as a template, *in vitro* RNA amplification was performed using T7 MEGAscript (Ambion) kit, the amplified RNA was purified using RNeasy mini kit, and the purified RNA was labeled with Cy3 and Cy5 (Cat # PA13105, GE Healthcare Bioscience, Pittsburgh, PA, US), and then purified using RNeasy mini Kit (Cat # 74106, QIAGEN, GmBH, Germany). Specific details can be found in operation specification.

**Chip hybridization**

Then, chip hybridization was performed based on the amount (Cat # 5188-5242, Agilent technologies, Santa Clara, CA, US) in Hybridization Oven (Cat # G 2545A, Agilent technologies, Santa Clara, CA, US). After 17 hours, the chip was washed (Cat # 121, Thermo Shandon, Waltham, MA, US) (Cat # 5188-5327, Agilent technologies, Santa Clara, CA, US), fixed, and dried (Cat # 121, Thermo Shandon, Waltham, MA, US) with Gene Expression Wash Buffer Kit (Cat # 5188-5327, Agilent technologies, Santa Clara, CA, US). All of operations can refer to operation specification. The same probe signals were combined and averaged. For each ORF, the sum of signal values in 8 sections was normalized to 1, and then PSI and STDEV values were calculated. Specific details for the calculation can be found in literatures (Hsueh-Chi Sherry Yen, et al., 2008, science).

**Search for important *in vivo* biological pathways and key proteins, the stability of which is affected upon Bortezomib treatment**

For finding Bortezomib-sensitive protein, ORFs-mEGFP$_{fu}$-mRFP$_f$ cell library is divided into two groups: for control group, DMSO was added; and for experimental group, Bortezomib was added (1 μM final concentration). And then cells were sorted by FACS in 8 sections, and the effects of Bortezomib treatment on the stability of genome-wide proteins were detected.

Data were deeply analyzed by system biology.

**GO annotation and analysis**

For each ORF, change in PSI was calculated by the inventors under DMSO and Bortezomib treatment conditions. ORFs were ranked according to ΔPSI / PSI by the inventors, and then GO enrichment analysis was applied to the top 15% of genes in the rank. All of the probes on the chip were used as control genes, and DAVID online software was used for GO enrichment analysis (Glynn Dennis Jr, 2003; da Wei Huang, 2009) by the inventors.

**Signaling pathway enrichment analysis**

DAVID online software was used to search 14 functional annotation databases, such as Kyoto Encyclopedia of Genes and Genomes (KEGG) and GO annotation, Biocarta pathway, BBID, and PANTHER pathway, for signaling pathways, the significance of which is changed.

**Protein-protein interaction network**

For better understanding overall effects on cell signaling networks upon the addition of Bortezomib, protein-protein interaction network analysis was applied to the top 5% of genes in ΔPSI/PSI rank selected by the inventors, and online tool STRING database (Snel B, 2000) was used.

**Small compound-protein interaction analysis (CPI)**

Protein-small compound interaction network is important for understanding the function of molecules and cells. Compound-protein interaction network was analyzed using STITCH ('search tool for interactions of chemicals') (Kuhn et al., 2008) (2.0 version) (http://stitch2.embl.de/) online database.

**MTT detection of cell viability**

The concentration of the cell suspension was adjusted, the cells were plated at a density of 1000-10000 cells to be tested / well, a concentration gradient of Bortezomib, 17-AAG (Selleck Chemicals) or C75 (Cayman Chemical) was added, 4-6 replicates for each concentration. Plates were incubated for 24-72 hours. To each well was added 20 ul MTT (3-(4,5-dimethyl-2-thiazole)-2,5-diphenyl tetrazolium bromide) solution, culture was continued for another 4 hrs. To each well was added 150 ul of dimethyl sulfoxide, shaken on a shaker at low speed for 10 mins for thoroughly dissolving the crystals. Absorbance of each well was measured on an enzyme-linked immune detector at OD 490 nm (570 nm).

**Interaction parameter CI (Combination index) analysis**

CompuSyn software (http://www.combosyn.com/) was used to analyze BTZ and 17-AAG or C75 interaction mode. Chou-Talalay principle was applied to interaction equation (Chou TC, P Talalay, Quantitative Analysis of dose-Effect relationships: the Combined Effects of Multiple Drugs or Enzyme inhibitors. Advances in Enzyme Regulation 02/1984; 22: 27-55), that is, when CI = 1, > 1, or <1, the drug can be deemed as exhibiting additional, antagonistic or synergistic effect, respectively. CZ-1 cells were added into 96-well plate at 5,000 cells per well, and corresponding concentrations of BTZ were added respectively in combination with 17-AAG or C75, and after 24 hr, cell viability was detected using MTT.

**Example 1. Efficient detection of relative stability of a protein by dual-fluorescent protein system**

A dual-fluorescent protein structure was designed by the inventors, wherein mEGFP was fused to C-terminal of target protein, intermediate linker protein is ubiquitin with K(R) saturated mutation, and internal reference fluorescent protein is mRFP, thereby forming a structure of $mEGFP_{fu}$-$mRFP_f$, and wherein mEGFP is of the sequence of SEQ ID NOs: 1 and 2, mRFP is of the sequence of SEQ ID NO: 3, 4, $Ub_{K0}$ is of the sequence of SEQ ID NOs: 17 and 18. A schematic view of the structure is shown in Figure 1a, the principle for the relative quantification of target proteins is shown in FIG 1b. Artificially designed recombinant dual-fluorescent protein structure was constructed on an expression plasmid of mammalian cell by the inventors, and constitutively expressed by using CMV promoter.

$mEGFP_{fu}$-$mRFP_f$ without any exogenous gene being fused was constructed into an eukaryotic expression vector, and 293 FT cells were infected through lentivirus, thereby successfully constructing a stably transfected cell line with $mEGFP_{fu}$-$mRFP_f$ being fused. Through FACS, mEGFP-$UB_{K0}$ was detected as having the same stability as mRFP, both of which are very stable. See Figure 1C, wherein cells in low signal area are negative control cells.

For detecting the relative stability of a gene, gene ARC, TP53, CDC 25A were fused to N-terminal of mEGFP in mEGFP-$UB_{K0}$-mRFP structure through gateway recombinant technology. 293 FT cells were infected through lentivirus, thereby successfully constructing a stably transfected cell line with ARC-$mEGFP_{fu}$-$mRFP_f$, TP53-$mEGFP_{fu}$-$mRFP_f$, CDC25A-$mEGFP_{fu}$-$mRFP_f$ being fused. Through FACS, the inventors found that different genes correspond to different protein stabilities. See Figure 5.

For verifying whether the dual-fluorescent protein will be cleaved by deubiquitinating enzyme, the inventors tested the protein expressed by TP53-$mEGFP_{fu}$-$mRFP_f$ vector as an example. The original band of the fusion peptide which has just been translated, and two small bands (TP53-mEGFP fusion protein band and mRFP band) formed through cleavage with ubiquitination enzyme were verified through western blot (see Fig. 7). It demonstrates that the fusion polypeptide provided in the present invention was successfully cleaved by ubiquitination enzymes, and two protein fragments are formed as expected.

For testing whether the dual-fluorescent protein structure can monitor the effects of exogenous stimulus or disturbance on protein stability in real-time, AXNA1-$mEGFP_{fu}$-$mRFP_f$ and RGS4-$mEGFP_{fu}$-$mRFP_f$ cell lines were constructed by the inventors, and the response of the cell line toward anti-cancer drug Bortezomib was detected. It is demonstrated that changes in protein stability can be sensitively and specifically detected by the dual-fluorescent system of the present invention, based on FACS and western blotting detection results. See Figure 1d.

**Example 2. Genome-wide detection of protein stability by mEGFP-$UB_{K0}$-mRFP dual-fluorescent protein system**

C-termini of about 15000 ORFs were fused to mEGFP-$UB_{K0}$-mRFP structure through gateway recombinant technology by using human ORFeome V5.1 as target gene library. 293 cells were infected through lentivirus infection by using a gene library with $mEGFP_{fu}$-$mRFP_f$ being fused, thereby constructing a dual-fluorescent cell line with library genes being integrated. See Figure 6. And then the library was sorted into 8 sections based on the ratio of mEGFP / mRFP by FACS technology. Genomic DNA from cells in each section was extracted, *in vitro* cRNA was amplified with PCR products as templates, and then the product was subjected to chip hybridization. Data were processed by using Composite loess normalization program in Bioconductor package limma, and relative stability parameter PSI (Protein Stability Index) for each gene was calculated. The flow chart can be found in Figure 2a. The distribution of relative stability parameter of protein can be found in Figure 2b, and representative data can be found in Figure 2c, d. Figure 2d is a broken line graph plotted based on the data shown in Figure 2c, wherein responsive genes are distributed as single peaks.

**Example 3. Detection of candidate genes responsive to anti-cancer drug Bortezomib at protein stability level by dual-fluorescent cell library**

DMSO (control treatment) and Bortezomib (experimental treatment) were used to treat cells in dual-fluorescent cell library containing human ORFeome V5.1 gene library, respectively, by the inventors. And then according to the scheme shown in Figure 2a, PSI values for each library gene under DMSO and Bortezomib were calculated, $\Delta PSI = PSI_{BTZ}-PSI_{DMSO}$ was obtained, all of the genes were ranked based on ΔPSI and significant genes were analyzed through bioinformatics. Figure 3a shows the chemical structure of anticancer drug Bortezomib; Figure 3c, d are representative data and substrate-responsive proteins; and Figure 4 is bioinformatics analysis results.

**Example 4. Guide to rational drug combination from CPI analysis of ProTA data**

Protein-small compound interaction network is important for understanding the function of molecules and cells. Therefore, the inventors have studied small compounds significantly interacting with BTZ effectors using STITCH (search tool for interactions of chemicals) online database for exploring cellular mechanisms between the cytotoxicity of Bortezomib and the development of drug resistance. DMSO (control treatment) and Bortezomib (experimental treatment) were used to treat cells in the library obtained in Example 2, respectively. And then according to the scheme shown in Figure 2a, PSI values for each library gene under DMSO and Bortezomib were calculated, $\Delta PSI = PSI_{BTZ}-PSI_{DMSO}$ was obtained, all of the genes were ranked based on ΔPSI and CPI (compound-protein interaction) analysis was applied to the top 250 hits. CPI analysis results are shown in Figure 8. From CPI analysis results, it is demonstrated that small compounds adenosine diph (replaced by analog 17-AAG) and coenzyme A (using FASN inhibitor C75) were specifically enriched by the inventors.

Subsequently, the inventors have verified the effects of BTZ in combination with 17-AAG or C75 by using CZ-1 and CZ-1 / R cell line as verification cell line, and interaction parameter CI (Combination index) was calculated, that is, interaction mode of drug BTZ with 17-AAG or C75 was analyzed, and the results are shown in Figure 9 and 10. Figure 9, showing the effects of BTZ in combination with 17-AAG or C75 detected by MTT, shows that BTZ in combination with 17-AAG or C75 exhibits significant killing effects on BTZ sensitive cell line CZ-1 or BTZ-resistance CZ-1 / R cell line. Figure 10 shows that BTZ at concentration of 5, 10, 15 and 20 nM exhibits synergistic effects with 17-AAG, and BTZ at all of the tested concentrations exhibits synergistic effects with C75.

## Claims

**1.** A genetic construct with a structure of the following formula:

$$5'\text{-}A + B + C + D + E\text{-}3',$$

wherein,

A represents a promoter;
B represents a coding sequence for a fusion protein consisting of a target protein and a first marker protein;
C represents a coding sequence for a linker peptide;
D represents a coding sequence for a second marker protein; and
E represents a terminator;
wherein B and D can be interchanged;
B, from 5' to 3', comprises a coding sequence for the target protein and a coding sequence for the first marker protein;
the coding sequence of the target protein is any gene selected from human genomic library human ORFeome V5.1;
the linker peptide is ubiquitin, or ubiquitin with K(R) saturation mutation;
the first marker protein and the second marker protein are fluorescent proteins; and
wherein the target protein, first marker protein, linker peptide and second marker protein are co-expressed under the same promoter; and
and wherein the genetic construct encodes a polypeptide comprising successively the target protein, the first marker protein, linker peptide and the second marker protein, or the second marker protein, the linker peptide, the target protein and the first marker protein.

**2.** A polypeptide with the structure of a following formula:

$$B1 + C1 + D1,$$

wherein,

B1 represents a fusion protein consisting of a target protein and a first marker protein;
C1 represents a linker peptide; and
D1 represents a second marker protein;
wherein B1 and D1 can be interchanged;
the polypeptide is encoded by genetic construct of claim 1;
the linker peptide is ubiquitin or ubiquitin with K(R) saturation mutation; and
the first marker protein and the second marker protein are fluorescent proteins,
wherein the polypeptide comprises successively the target protein, the first marker protein, linker peptide and the second marker protein, or the second marker protein, the linker peptide, the target protein and the first marker protein.

**3.** A vector comprising the genetic construct according to claim 1.

**4.** A cell comprising the genetic construct according to claim 1 or the vector according to claim 3.

**5.** A cell library consisting of the cells according to claim 4.

**6.** A method for detecting the stability of one or more target proteins, comprising the following steps:

(1) constructing the cell library according to claim 5;
(2) culturing the cell library obtained in (1) under specific conditions and control conditions;
(3) determining the ratio of the first marker protein to the second marker protein in the cell library cultured under the specific conditions and control conditions; and
(4) drawing a conclusion that the stability of the target protein is changed or not, based on the ratio of the first marker protein to the second marker protein in the cell line cultured under the specific conditions and control conditions; or:
based on the ratio of the first marker protein to the second marker protein, separating the cell into n sections ($n \geq 2$) through flow cytometry, detecting the expression of each target gene in each section by DNA chip technology to calculate the distribution of the target genes in each section, and comparing the distributions of target genes in cell lines cultured under the specific conditions and control conditions, thereby drawing a conclusion that the stability of the protein encoded by the target gene is changed or not.

**Patentansprüche**

1. Genetisches Konstrukt mit einer Struktur der nachstehenden Formel:

$$5'\text{-}A + B + C + D + E\text{-}3',$$

worin

A für einen Promotor steht;
B für eine Kodierungssequenz für ein Fusionsprotein steht, das aus einem Zielprotein und einem ersten Marker-Protein besteht;
C für eine Kodierungssequenz für ein Linkerpeptid steht;
D für eine Kodierungssequenz für ein zweites Marker-Protein steht; und
E für einen Terminator steht;
wobei B und D ausgetauscht werden können;
B von 5' zu 3' eine Kodierungssequenz für das Zielprotein und eine Kodierungssequenz für das erste Marker-Protein umfasst;
die Kodierungssequenz des Zielproteins ein beliebiges Gen ist, das aus der menschlichen Genombibliothek Human ORFeome V5.1 ausgewählt ist;
das Linkerpeptid Ubiquitin oder Ubiquitin mit K(R)-Sättigungsmutation ist;
das erste Marker-Protein und das zweite Marker-Protein fluoreszierende Proteine sind; und
wobei das Zielprotein, das erste Marker-Protein, das Linkerpeptid und das zweite Marker-Protein unter demselben Promotor gemeinsam exprimiert werden; und
wobei das genetische Konstrukt für ein Polypeptid kodiert, das aufeinanderfolgend das Zielprotein, das erste Marker-Protein, das Linkerpeptid und das zweite Marker-Protein oder das zweite Marker-Protein, das Linkerpeptid, das Zielprotein und das erste Marker-Protein umfasst.

2. Polypeptid mit der Struktur der nachstehenden Formel:

$$B1 + C1 + D1,$$

worin

B1 für ein Fusionsprotein steht, das aus einem Zielprotein und einem ersten Marker-Protein besteht;
C1 für ein Linkerpeptid steht und
D1 für ein zweites Marker-Protein steht;
wobei B1 und D1 ausgetauscht werden können;
das Polypeptid durch ein genetisches Konstrukt nach Anspruch 1 kodiert wird;
das Linker-Peptid Ubiquitin oder Ubiquitin mit K(R)-Sättigungsmutation ist; und
das erste Marker-Protein und das zweite Marker-Protein fluoreszierende Proteine sind;
wobei das Polypeptid aufeinanderfolgend das Zielprotein, das erste Marker-Protein, das Linkerpeptid und das zweite Marker-Protein oder das zweite Marker-Protein, das Linker-peptid, das Zielprotein und das erste Marker-Protein umfasst.

3. Vektor, der ein genetisches Konstrukt nach Anspruch 1 umfasst.

4. Zelle, die ein genetisches Konstrukt nach Anspruch 1 oder einen Vektor nach Anspruch 3 umfasst.

5. Zellbibliothek, die aus Zellen nach Anspruch 4 besteht.

6. Verfahren zur Detektion der Stabilität eines oder mehrerer Zielproteine, wobei das Verfahren die folgenden Schritte umfasst:

(1) das Konstruieren einer Zellbibliothek nach Anspruch 5;
(2) das Kultivieren der in (1) erhaltenen Zellbibliothek unter spezifischen Bedingungen und Kontrollbedingungen;
(3) das Bestimmen des Verhältnisses des ersten Marker-Proteins zu dem zweiten Marker-Protein in der Zellbibliothek, die unter den spezifischen Bedingungen und den Kontrollbedingungen kultiviert wurde; und
(4) das Schlussfolgern, dass die Stabilität des Zielproteins verändert ist oder nicht, auf Grundlage des Verhältnisses des ersten Marker-Proteins zu dem zweiten Marker-Protein in der unter den spezifischen Bedingungen und Kontrollbedingungen kultivierten Zelllinie; oder

auf Grundlage des Verhältnisses des ersten Marker-Proteins zu dem zweiten Marker-Protein das Aufteilen der Zellen in n Abschnitte ($n \geq 2$) mittels Durchflusszytometrie, das Detektieren der Expression jedes Zielgens in jedem Abschnitt mittels DNA-Chip-Technologie zur Berechnung der Verteilung der Zielgene in jedem Abschnitt und das Vergleichen der Verteilung der Zielgene in Zelllinien, die unter spezifischen Bedingungen und Kontrollbedingungen kultiviert wurden, und anhand dessen das Schlussfolgern, dass die Stabilität des durch das Zielgen kodierten Proteins verändert ist oder nicht.

## Revendications

**1.** Construction génétique ayant une structure de la formule suivante :

$$5'\text{-}A+B+C+D+E\text{-}3',$$

dans laquelle,

A représente un promoteur ;
B représente une séquence codante pour une protéine de fusion constituée d'une protéine cible et d'une première protéine marqueur ;
C représente une séquence codante pour un peptide lieur ;
D représente une séquence codante pour une seconde protéine marqueur ; et
E représente un terminateur ;
dans laquelle B et D peuvent être interchangés ;
B, de 5' vers 3', comprend une séquence codante pour la protéine cible et une séquence codante pour la première protéine marqueur ;
la séquence codante de la protéine cible est tout gène choisi dans la banque génomique humaine d'ORFeome V5.1 humain ;
le peptide lieur est l'ubiquitine ou l'ubiquitine avec une mutation de saturation K(R) ;
la première protéine marqueur et la seconde protéine marqueur sont des protéines fluorescentes ;
et
dans laquelle la protéine cible, la première protéine marqueur, le peptide lieur et la seconde protéine marqueur sont co-exprimés sous le même promoteur ; et
et dans lequel la construction génétique code pour un polypeptide comprenant successivement la protéine cible, la première protéine marqueur, le peptide lieur et la seconde protéine marqueur, ou la seconde protéine marqueur, le peptide lieur, la protéine cible et la première protéine marqueur.

**2.** Polypeptide ayant la structure d'une formule suivante :

$$B1+C1+D1,$$

dans lequel,

B1 représente une protéine de fusion constituée d'une protéine cible et d'une première protéine marqueur ;
C1 représente un peptide lieur ; et
D1 représente une seconde protéine marqueur ;
dans lequel B1 et D1 peuvent être interchangés ;
le polypeptide est codé par une construction génétique de la revendication 1 ;
le peptide lieur est l'ubiquitine ou l'ubiquitine avec une mutation de saturation K(R) ; et
la première protéine marqueur et la seconde protéine marqueur sont des protéines fluorescentes,
dans lequel le polypeptide comprend successivement la protéine cible, la première protéine marqueur, le peptide lieur et la seconde protéine marqueur, ou la seconde protéine marqueur, le peptide lieur, la protéine cible et la première protéine marqueur.

3. Vecteur comprenant la construction génétique selon la revendication 1.

4. Cellule comprenant la construction génétique selon la revendication 1 ou le vecteur selon la revendication 3.

5. Bibliothèque de cellules constituée des cellules selon la revendication 4.

6. Procédé de détection de la stabilité d'une ou plusieurs protéines cibles, comprenant les étapes suivantes consistant à :

(1) construire la bibliothèque de cellules selon la revendication 5 ;
(2) mettre en culture la banque de cellules obtenue en (1) dans des conditions spécifiques et des conditions de contrôle ;
(3) déterminer le rapport de la première protéine marqueur à la seconde protéine marqueur dans la banque de cellules cultivées dans les conditions spécifiques et les conditions de contrôle ; et
(4) tirer une conclusion que la stabilité de la protéine cible est modifiée ou non, sur la base du rapport de la première protéine marqueur à la seconde protéine marqueur dans la lignée cellulaire cultivée dans les conditions spécifiques et les conditions de contrôle ; ou :
sur la base du rapport de la première protéine marqueur à la seconde protéine marqueur, séparer la cellule en n sections (n≥2) par cytométrie en flux, détecter l'expression de chaque gène cible dans chaque section par la technologie des puces à ADN pour calculer la distribution des gènes cibles dans chaque section, et comparer les distributions des gènes cibles dans des lignées cellulaires cultivées dans les conditions spécifiques et les conditions de contrôle, tirant ainsi une conclusion que la stabilité de la protéine codée par le gène cible est modifiée ou non.

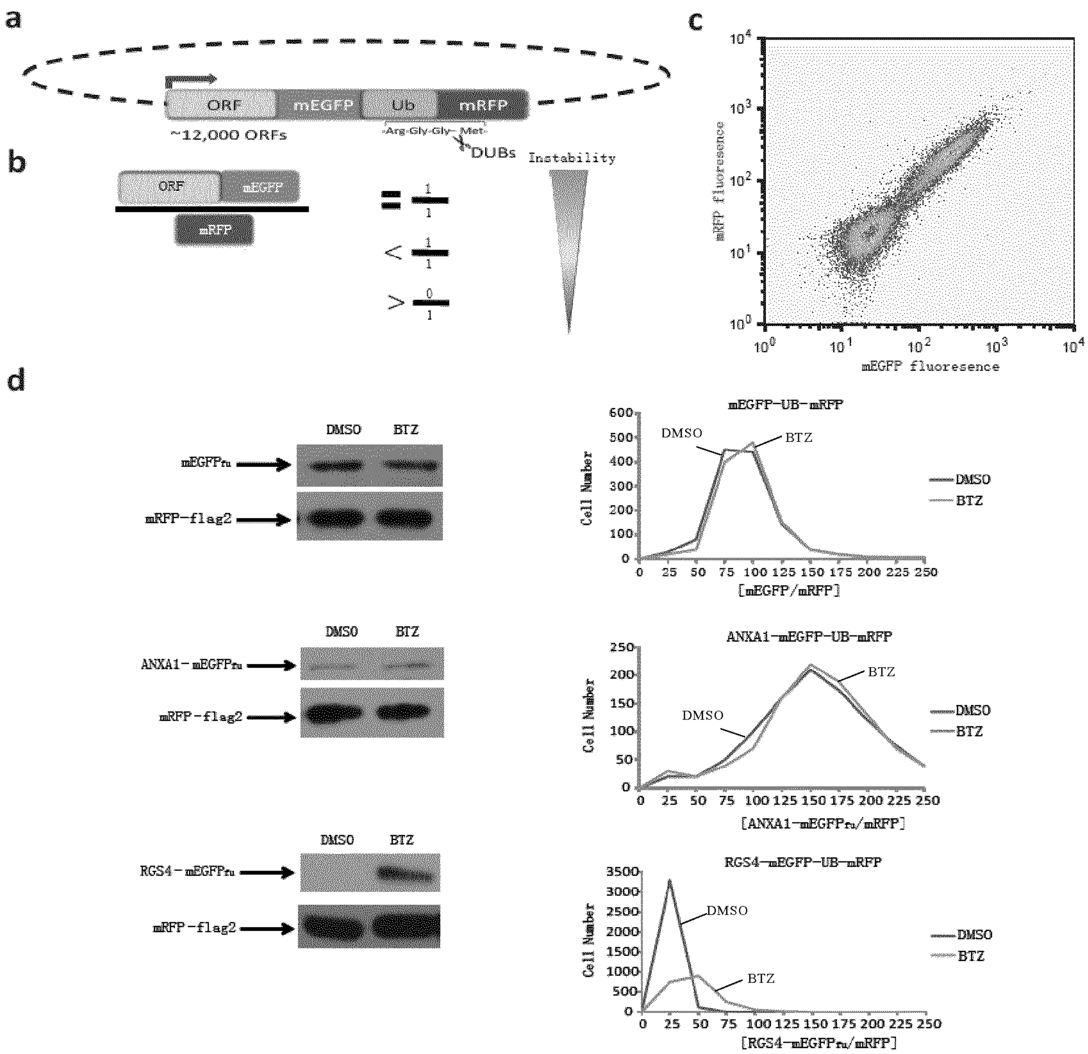
a
ORF
mEGFP
Ub
mRFP
~12,000 ORFs
-Arg-Gly-Gly-Met-
DUBs
b
ORF
mEGFP
mRFP
Instability
c
mRFP fluoresence
mEGFP fluoresence
d
DMSO
BTZ
mEGFPfu
mRFP-flag2
ANXA1-mEGFPfu
RGS4-mEGFPfu
mEGFP-UB-mRFP
ANXA1-mEGFP-UB-mRFP
RGS4-mEGFP-UB-mRFP
Cell Number
[mEGFP/mRFP]
[ANXA1-mEGFPfu/mRFP]
[RGS4-mEGFPfu/mRFP]

Fig. 1

a

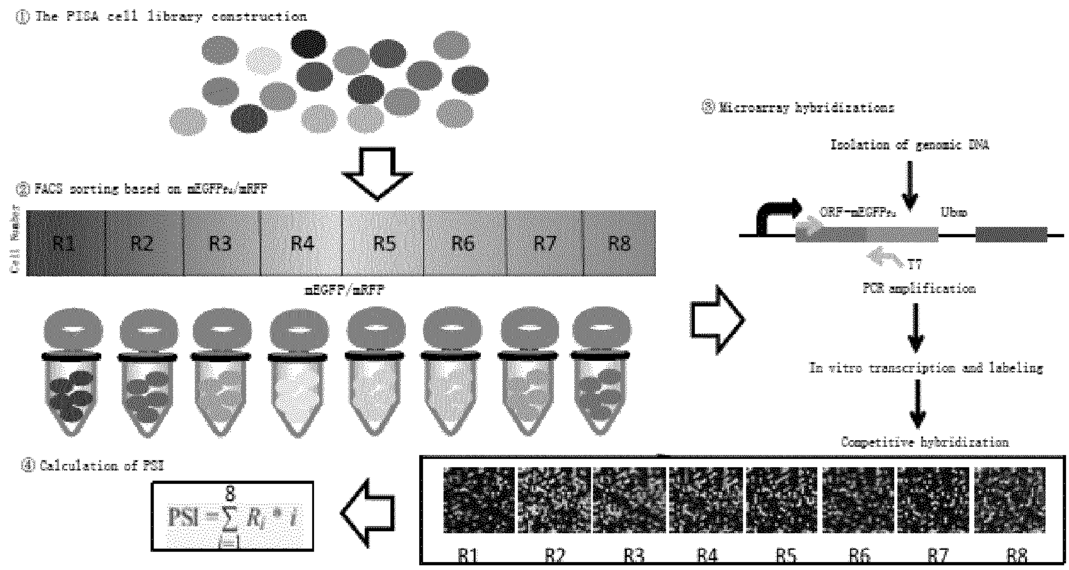


b

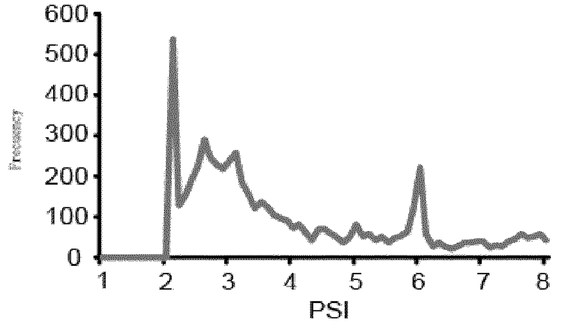


c

| Gene ID | R1 | R2 | R3 | R4 | R5 | R6 | R7 | R8 | SUM | PSI | STDEV |
|---|---|---|---|---|---|---|---|---|---|---|---|
| 80153 | 0.87 | 0.03 | 0.02 | 0.02 | 0.02 | 0.01 | 0.01 | 0.03 | 1.00 | 1.51 | 1.55 |
| 285533 | 0.00 | 0.79 | 0.21 | 0.00 | 0.00 | 0.00 | 0.00 | 0.00 | 1.00 | 2.21 | 0.42 |
| 255101 | 0.00 | 0.30 | 0.41 | 0.29 | 0.00 | 0.00 | 0.00 | 0.00 | 1.00 | 2.99 | 0.78 |
| 5082 | 0.00 | 0.00 | 0.22 | 0.67 | 0.00 | 0.05 | 0.00 | 0.06 | 1.00 | 4.10 | 1.15 |
| 2760 | 0.00 | 0.00 | 0.02 | 0.00 | 0.79 | 0.18 | 0.00 | 0.00 | 1.00 | 5.14 | 0.55 |
| 196051 | 0.00 | 0.00 | 0.03 | 0.00 | 0.37 | 0.59 | 0.00 | 0.00 | 1.00 | 5.53 | 0.71 |
| 440337 | 0.02 | 0.04 | 0.02 | 0.01 | 0.01 | 0.20 | 0.66 | 0.03 | 1.00 | 6.38 | 1.42 |

| |
|---|
| <9% |
| 10~19% |
| 20~29% |
| 30~39% |
| 40~49% |
| 50~59% |
| 60~69% |
| 70~79% |
| >80% |

d

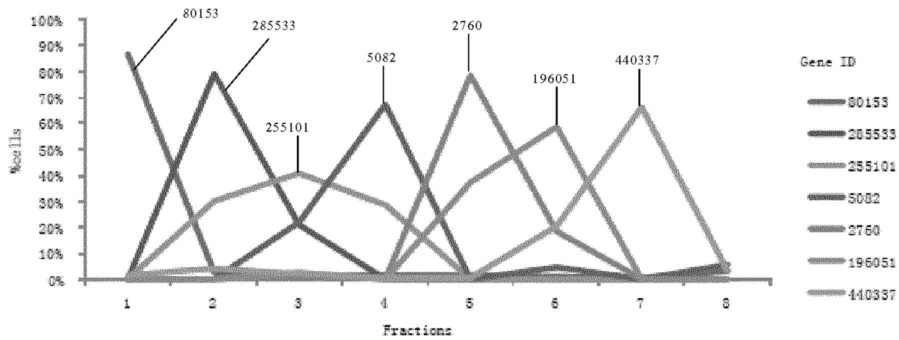


Fig. 2

a

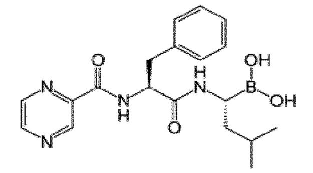

Bortezomib

b

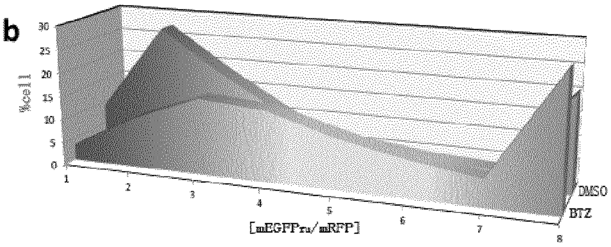


c

| Treatment | Gene ID | R1 | R2 | R3 | R4 | R5 | R6 | R7 | R8 | PSI | STDEV | △PSI | △PSI/PSI | Substrate? |
|---|---|---|---|---|---|---|---|---|---|---|---|---|---|---|
| DMSO | 80135 | 0.00 | 1.00 | 0.00 | 0.00 | 0.00 | 0.00 | 0.00 | 0.00 | 2.01 | 0.26 | | | |
| Bortezomib | 80135 | 0.00 | 0.00 | 0.00 | 0.00 | 0.00 | 0.00 | 0.00 | 1.00 | 7.98 | 0.32 | 5.97 | 2.97 | Yes |
| DMSO | 6892 | 0.01 | 0.97 | 0.01 | 0.00 | 0.00 | 0.00 | 0.00 | 0.01 | 2.10 | 0.75 | | | |
| Bortezomib | 6892 | 0.01 | 0.10 | 0.02 | 0.02 | 0.01 | 0.01 | 0.01 | 0.83 | 7.13 | 2.03 | 5.03 | 2.39 | Yes |
| DMSO | 1908 | 0.00 | 0.00 | 0.87 | 0.00 | 0.00 | 0.00 | 0.00 | 0.12 | 3.62 | 1.65 | | | |
| Bortezomib | 1908 | 0.00 | 0.00 | 0.03 | 0.00 | 0.00 | 0.00 | 0.20 | 0.77 | 7.63 | 0.95 | 4.01 | 1.11 | Yes |
| DMSO | 4221 | 0.01 | 0.98 | 0.00 | 0.00 | 0.00 | 0.00 | 0.00 | 0.00 | 2.04 | 0.48 | | | |
| Bortezomib | 4221 | 0.05 | 0.66 | 0.04 | 0.02 | 0.06 | 0.03 | 0.09 | 0.06 | 3.11 | 2.05 | 1.07 | 0.53 | No |
| DMSO | 57130 | 0.00 | 0.96 | 0.00 | 0.00 | 0.00 | 0.03 | 0.00 | 0.00 | 2.14 | 0.74 | | | |
| Bortezomib | 57130 | 0.00 | 0.94 | 0.01 | 0.00 | 0.00 | 0.03 | 0.00 | 0.01 | 2.22 | 0.98 | 0.08 | 0.04 | No |

d

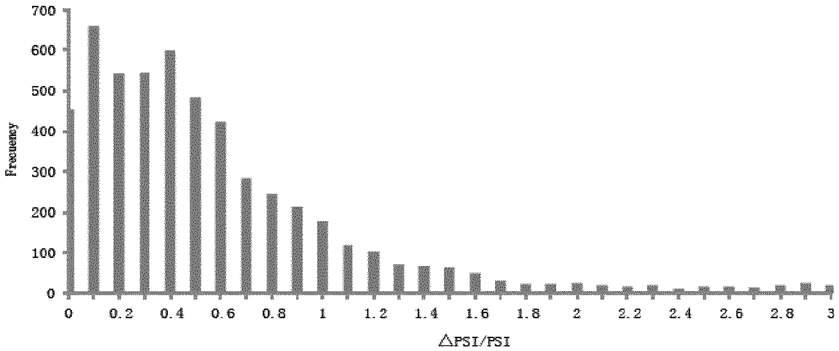


e

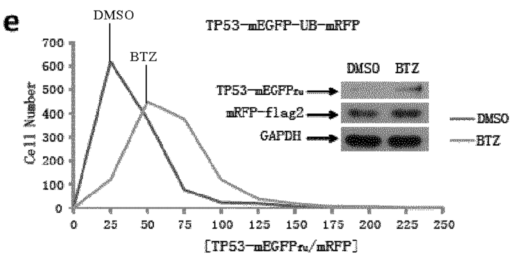


f

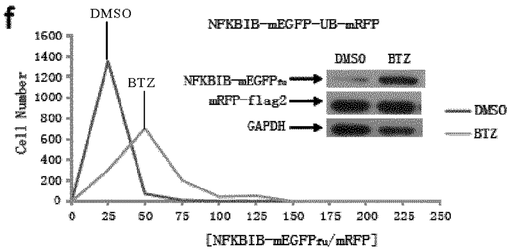


g

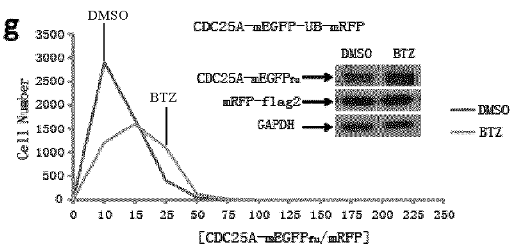


h

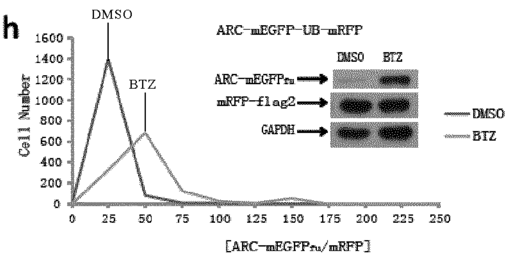


Fig. 3

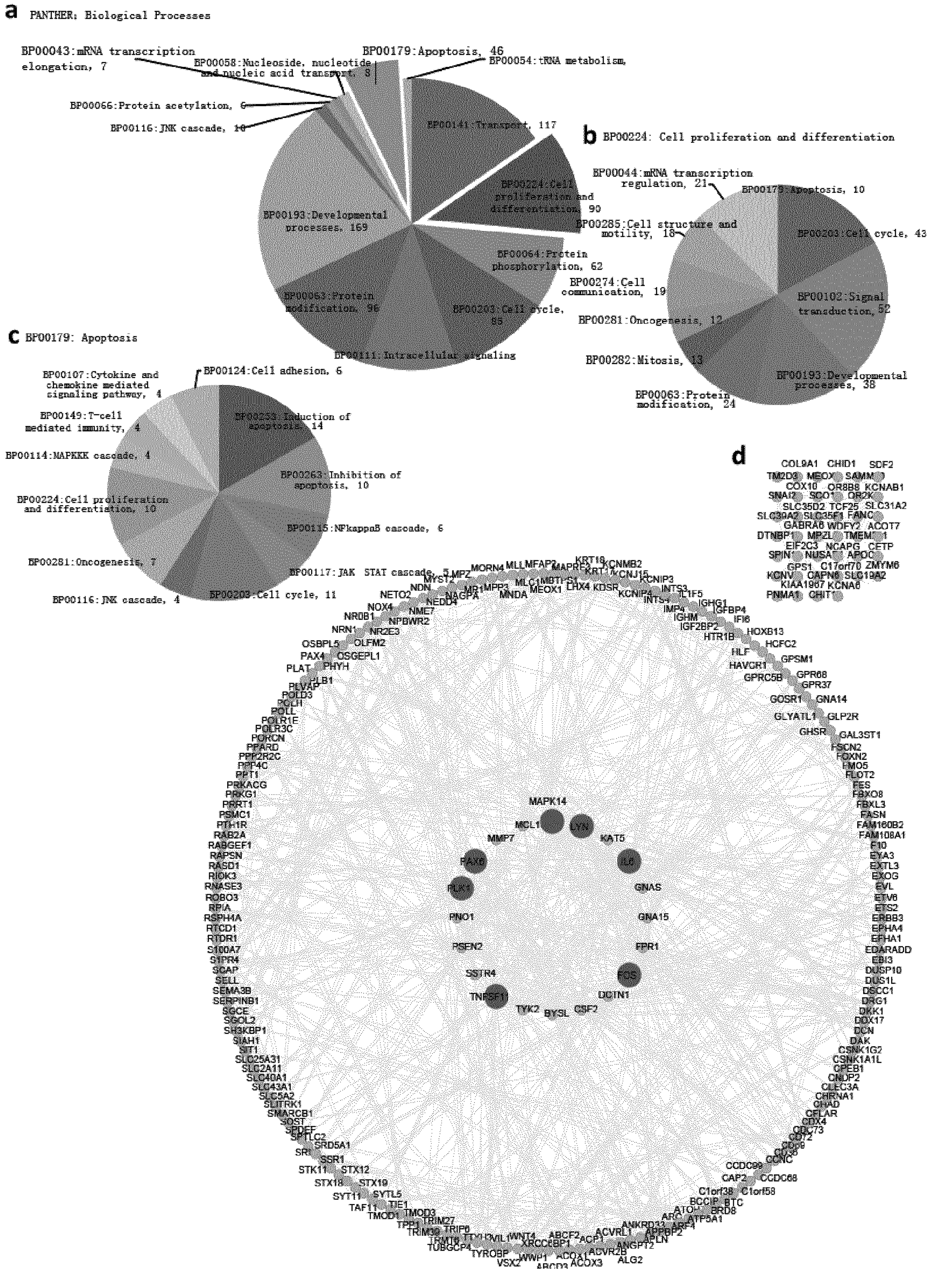

a PANTHER: Biological Processes
BP00043:mRNA transcription elongation, 7
BP00058:Nucleoside, nucleotide and nucleic acid transport, 8
BP00179:Apoptosis, 46
BP00054:tRNA metabolism,
BP00066:Protein acetylation, 6
BP00116:JNK cascade, 10
BP00141:Transport, 117
BP00224:Cell proliferation and differentiation, 90
BP00193:Developmental processes, 169
BP00064:Protein phosphorylation, 62
BP00063:Protein modification, 96
BP00203:Cell cycle, 85
BP00111:Intracellular signaling
b BP00224: Cell proliferation and differentiation
BP00044:mRNA transcription regulation, 21
BP00179:Apoptosis, 10
BP00285:Cell structure and motility, 18
BP00203:Cell cycle, 43
BP00274:Cell communication, 19
BP00102:Signal transduction, 52
BP00281:Oncogenesis, 12
BP00282:Mitosis, 13
BP00193:Developmental processes, 38
BP00063:Protein modification, 24
c BP00179: Apoptosis
BP00107:Cytokine and chemokine mediated signaling pathway, 4
BP00124:Cell adhesion, 6
BP00149:T-cell mediated immunity, 4
BP00253:Induction of apoptosis, 14
BP00114:MAPKKK cascade, 4
BP00263:Inhibition of apoptosis, 10
BP00224:Cell proliferation and differentiation, 10
BP00115:NFkappaB cascade, 6
BP00281:Oncogenesis, 7
BP00117:JAK STAT cascade, 5
BP00116:JNK cascade, 4
BP00203:Cell cycle, 11
d
MAPK14
MCL1
LYN
MMP7
KAT5
PAX6
IL6
PLK1
GNAS
PNO1
GNA15
PSEN2
FPR1
SSTR4
FOS
TNFSF11
DCTN1
TYK2
BYSL
CSF2


Fig. 4

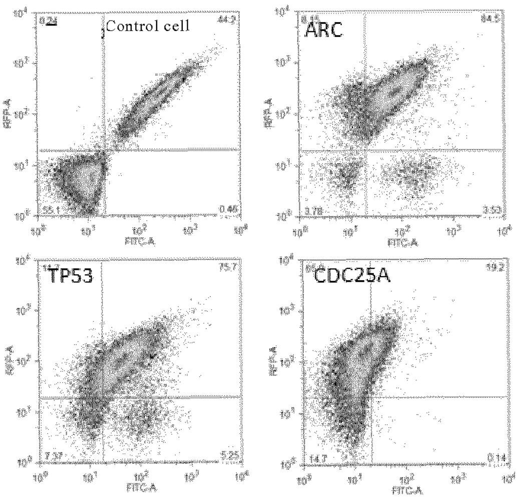


Fig. 5

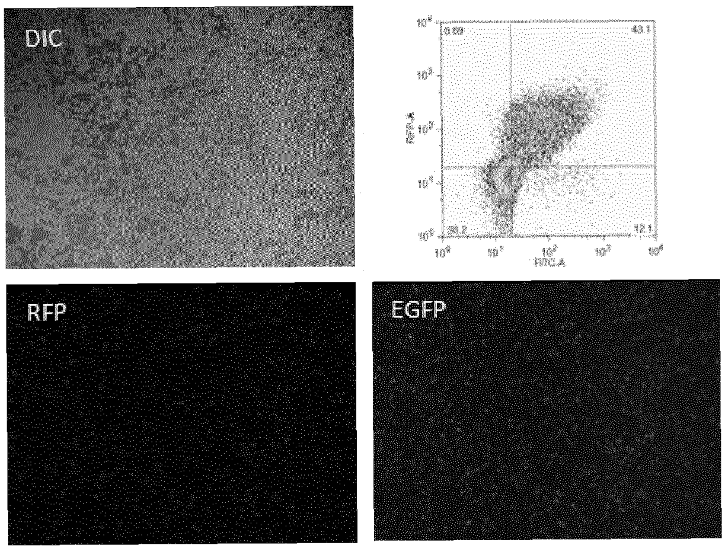


Fig. 6

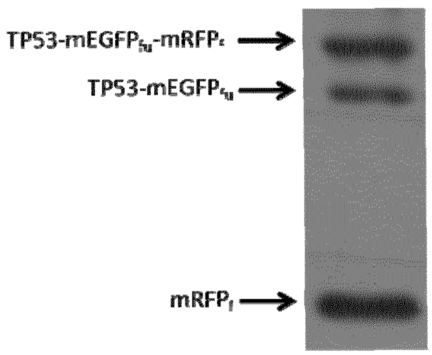


Fig. 7

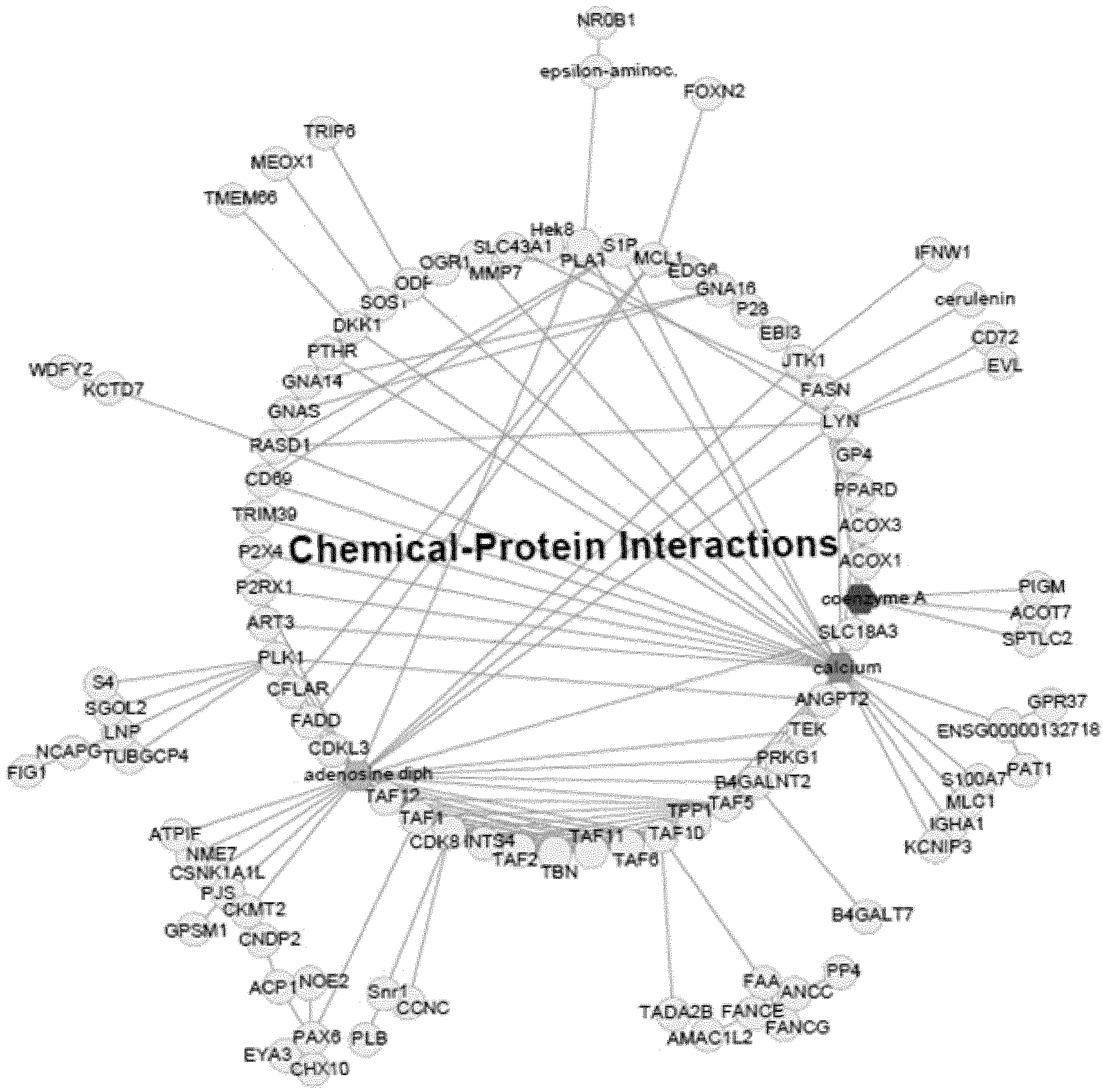
Chemical-Protein Interactions
NR0B1
epsilon-aminoc.
FOXN2
TRIP6
MEOX1
TMEM66
Hek8
SLC43A1
S1P
OGR1
MMP7
PLA1
MCL1
EDG6
GNA16
ODF
SOS1
DKK1
P28
EBI3
IFNW1
cerulenin
CD72
EVL
PTHR
JTK1
FASN
LYN
WDFY2
KCTD7
GNA14
GNAS
RASD1
GP4
PPARD
ACOX3
ACOX1
CD69
TRIM39
P2X4
P2RX1
ART3
coenzyme A
PIGM
ACOT7
SPTLC2
SLC18A3
calcium
PLK1
S4
SGOL2
LNP
NCAPG
TUBGCP4
FIG1
CFLAR
FADD
CDKL3
ANGPT2
TEK
GPR37
ENSG00000132718
PRKG1
B4GALNT2
adenosine diph
TAF12
TAF1
TAF5
TPP1
S100A7
PAT1
MLC1
IGHA1
KCNIP3
ATPIF
NME7
CSNK1A1L
PJS
CKMT2
GPSM1
CNDP2
CDK8
INTS4
TAF2
TBN
TAF11
TAF6
TAF10
B4GALT7
ACP1
NOE2
PAX6
EYA3
CHX10
Snr1
CCNC
PLB
TADA2B
AMAC1L2
FAA
FANCE
ANCC
PP4
FANCG

Fig. 8

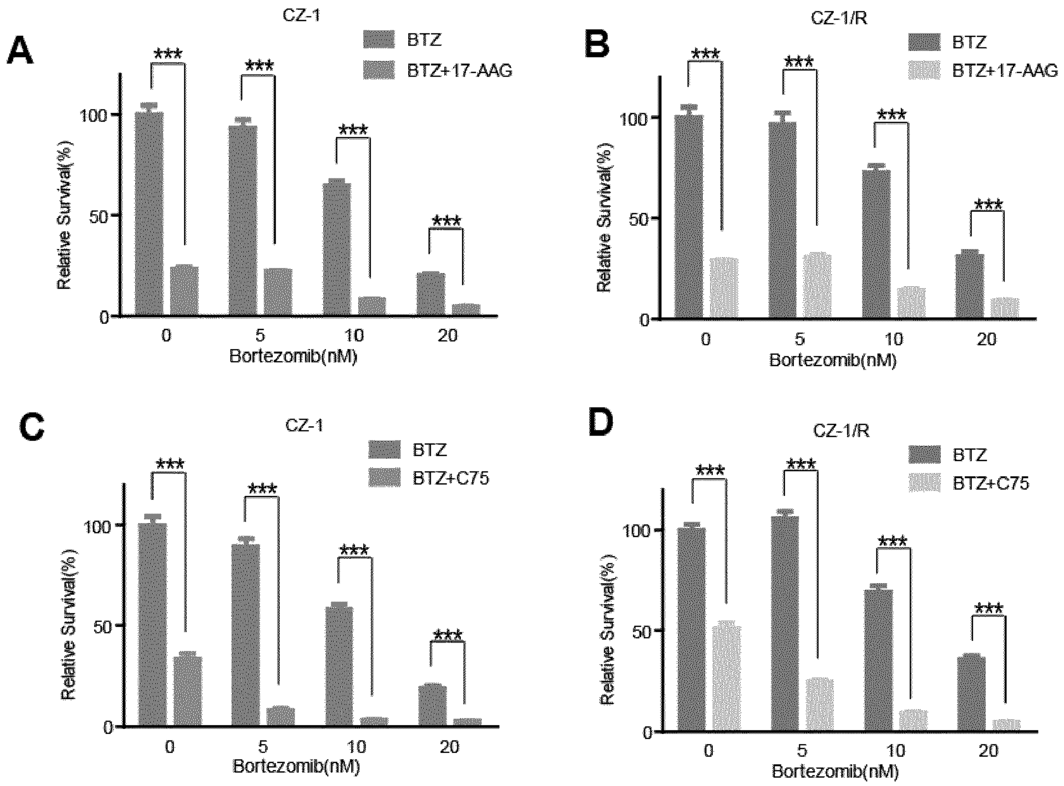


Fig. 9

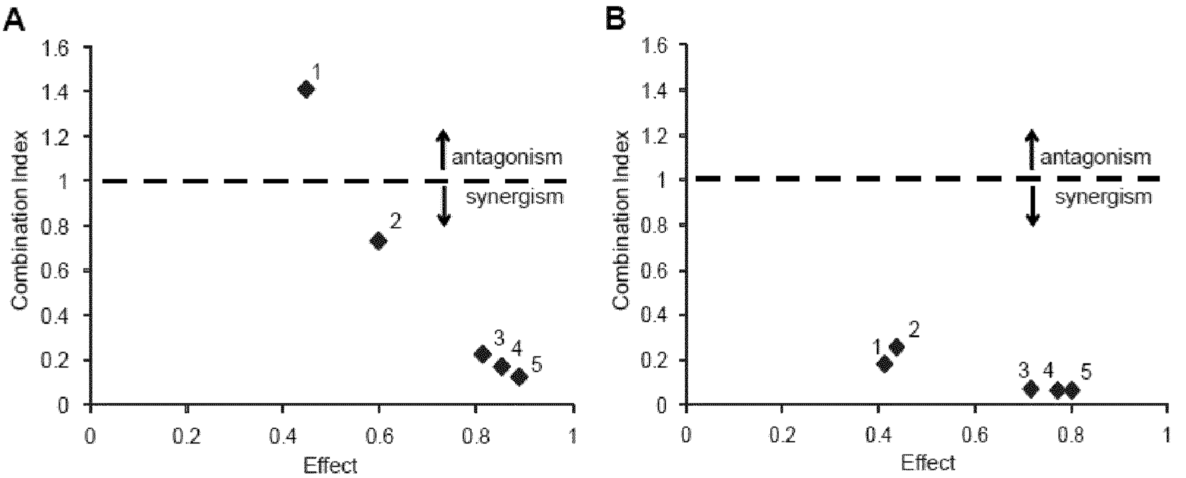


| Combination（CZ-1） | C75（ug/ml） | BTZ（nM） | CI |
|---|---|---|---|
| 1 | 10 | 1 | 1.414 |
| 2 | 10 | 5 | 0.732 |
| 3 | 10 | 10 | 0.223 |
| 4 | 10 | 15 | 0.168 |
| 5 | 10 | 20 | 0.121 |

| Combination（CZ-1） | 17-AAG（uM） | BTZ（nM） | CI |
|---|---|---|---|
| 1 | 50 | 1 | 0.195 |
| 2 | 50 | 5 | 0.270 |
| 3 | 50 | 10 | 0.084 |
| 4 | 50 | 15 | 0.077 |
| 5 | 50 | 20 | 0.078 |

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201310128492


### Non-patent literature cited in the description


- *Science,* 1984, vol. 224, 1431
- **SAMBROOK et al.** Molecular Cloning: A Laboratory Manual. Cold Spring Harbor Laboratory Press, 1989
- **HOU J ; LIN FY ; ZHANG B ; ZHANG LZ ; DING SQ.** Establishment and biological characteristics of human multiple myeloma cell line CZ-1. *Chinese Medical Journal,* 2004, vol. 117 (1), 115-119
- **HSUEH-CHI SHERRY YEN et al.** *science,* 2008
- **KUHN et al.** *search tool for interactions of chemicals,* 2008, http://stitch2.embl.de/
- **CHOU TC ; P TALALAY.** Quantitative Analysis of dose-Effect relationships: the Combined Effects of Multiple Drugs or Enzyme inhibitors. *Advances in Enzyme Regulation,* February 1984, vol. 22, 27-55

SEQUENCE LISTING

<110> SHANGHAI INSTITUTES FOR BIOLOGICAL SCIENCES, CHINESE ACADEMY OF SCIENCES

<120> Method for detecting protein stability and uses thereof

<130> P2014-0401

<150> CN201310128492.8
<151> 2013-04-12

<160> 18

<170> PatentIn version 3.5

<210> 1
<211> 717
<212> DNA
<213> Artificial sequence

<220>
<223> oligonucleotide

<400> 1

```
atggtgagca agggcgagga gctgttcacc ggggtggtgc ccatcctggt cgagctggac      60
ggcgacgtaa acggccacaa gttcagcgtg tccggcgagg gcgagggcga tgccacctac     120
ggcaagctga ccctgaagtt catctgcacc accggcaagc tgcccgtgcc ctggcccacc     180
ctcgtgacca ccctgaccta cggcgtgcag tgcttcagcc gctaccccga ccacatgaag     240
cagcacgact tcttcaagtc cgccatgccc gaaggctacg tccaggagcg caccatcttc     300
ttcaaggacg acggcaacta caagacccgc gccgaggtga agttcgaggg cgacaccctg     360
gtgaaccgca tcgagctgaa gggcatcgac ttcaaggagg acggcaacat cctggggcac     420
aagctggagt acaactacaa cagccacaac gtctatatca tggccgacaa gcagaagaac     480
ggcatcaagg tgaacttcaa gatccgccac aacatcgagg acggcagcgt gcagctcgcc     540
gaccactacc agcagaacac ccccatcggc gacggccccg tgctgctgcc cgacaaccac     600
tacctgagca cccagtccgc cctgagcaaa gaccccaacg agaagcgcga tcacatggtc     660
ctgctggagt tcgtgaccgc cgccgggatc actctcggca tggacgagct gtacaag        717
```

<210> 2
<211> 239
<212> PRT
<213> Artificial sequence

<220>
<223> polypeptide

<400> 2

```
Met Val Ser Lys Gly Glu Glu Leu Phe Thr Gly Val Val Pro Ile Leu
1               5                   10                  15
Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Thr Tyr Gly Val Gln Cys Phe Ser Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
```

```
                115                 120                 125
Ile Asp Phe Lys Glu Asp Gly Asn Ile Leu Gly His Lys Leu Glu Tyr
        130                 135                 140
Asn Tyr Asn Ser His Asn Val Tyr Ile Met Ala Asp Lys Gln Lys Asn
145                 150                 155                 160
Gly Ile Lys Val Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Lys Leu
        195                 200                 205
Ser Lys Asp Pro Asn Glu Lys Arg Asp His Met Val Leu Leu Glu Phe
    210                 215                 220
Val Thr Ala Ala Gly Ile Thr Leu Gly Met Asp Glu Leu Tyr Lys
225                 230                 235
```

<210> 3
<211> 675
<212> DNA
<213> Artificial sequence

<220>
<223> oligonucleotide

<400> 3

```
atggcctcct ccgaggacgt catcaaggag ttcatgcgct tcaaggtgcg catggagggc       60
tccgtgaacg gccacgagtt cgagatcgag ggcgagggcg agggccgccc ctacgagggc      120
acccagaccg ccaagctgaa ggtgaccaag ggcggccccc tgcccttcgc ctgggacatc      180
ctgtcccctc agttccagta cggctccaag gcctacgtga agcaccccgc cgacatcccc      240
gactacttga agctgtcctt ccccgagggc ttcaagtggg agcgcgtgat gaacttcgag      300
gacggcggcg tggtgaccgt gacccaggac tcctccctgc aggacggcga gttcatctac      360
aaggtgaagc tgcgcggcac caacttcccc tccgacggcc ccgtaatgca gaagaagacc      420
atgggctggg aggcctccac cgagcggatg taccccgagg acggcgccct gaagggcgag      480
atcaagatga ggctgaagct gaaggacggc ggccactacg acgccgaggt caagaccacc      540
tacatggcca agaagcccgt gcagctgccc ggcgcctaca agaccgacat caagctggac      600
atcacctccc acaacgagga ctacaccatc gtggaacagt acgagcgcgc cgagggccgc      660
cactccaccg gcgcc                                                       675
```

<210> 4
<211> 225
<212> PRT
<213> Artificial sequence

<220>
<223> polypeptide

<400> 4

```
Met Ala Ser Ser Glu Asp Val Ile Lys Glu Phe Met Arg Phe Lys Val
1               5                   10                  15
Arg Met Glu Gly Ser Val Asn Gly His Glu Phe Glu Ile Glu Gly Glu
            20                  25                  30
Gly Glu Gly Arg Pro Tyr Glu Gly Thr Gln Thr Ala Lys Leu Lys Val
        35                  40                  45
Thr Lys Gly Gly Pro Leu Pro Phe Ala Trp Asp Ile Leu Ser Pro Gln
    50                  55                  60
Phe Gln Tyr Gly Ser Lys Ala Tyr Val Lys His Pro Ala Asp Ile Pro
65                  70                  75                  80
Asp Tyr Leu Lys Leu Ser Phe Pro Glu Gly Phe Lys Trp Glu Arg Val
                85                  90                  95
Met Asn Phe Glu Asp Gly Gly Val Val Thr Val Thr Gln Asp Ser Ser
            100                 105                 110

Leu Gln Asp Gly Glu Phe Ile Tyr Lys Val Lys Leu Arg Gly Thr Asn
        115                 120                 125
Phe Pro Ser Asp Gly Pro Val Met Gln Lys Lys Thr Met Gly Trp Glu
    130                 135                 140
Ala Ser Thr Glu Arg Met Tyr Pro Glu Asp Gly Ala Leu Lys Gly Glu
145                 150                 155                 160
Ile Lys Met Arg Leu Lys Leu Lys Asp Gly Gly His Tyr Asp Ala Glu
                165                 170                 175
Val Lys Thr Thr Tyr Met Ala Lys Lys Pro Val Gln Leu Pro Gly Ala
            180                 185                 190
Tyr Lys Thr Asp Ile Lys Leu Asp Ile Thr Ser His Asn Glu Asp Tyr
        195                 200                 205
Thr Ile Val Glu Gln Tyr Glu Arg Ala Glu Gly Arg His Ser Thr Gly
    210                 215                 220
Ala
225
```

<210> 5
<211> 30
<212> DNA
<213> Artificial sequence

<220>
<221> misc_feature
<223> Primer

<400> 5
acaaagctta atggtgagca agggcgagga 30

<210> 6
<211> 32
<212> DNA
<213> Artificial sequence

<220>
<221> misc_feature
<223> Primer

<400> 6
tggacgagct gtacaaggga tctgactaca ag 32

<210> 7
<211> 66
<212> DNA

<213> Artificial sequence

<220>
<221> misc_feature
<223> Primer

<400> 7

gggtctgact acaaggacga tgacgataag ggcggagact acaaggacga tgacgataag 60
ggctct 66

<210> 8
<211> 38
<212> DNA
<213> Artificial sequence

<220>
<221> misc_feature
<223> Primer

<400> 8
acgatgacga taagggctct atgcagatct ttgtgagg 38

<210> 9
<211> 100
<212> DNA
<213> Artificial sequence

<220>
<221> misc_feature
<223> Primer

<400> 9

atgcagatct ttgtgaggac cctcactggc agaaccatca cccttgaggt cgagcccagt 60
gacaccattg agaatgtcag agccagaatt caagacaggg 100

<210> 10
<211> 99
<212> DNA
<213> Artificial sequence

<220>
<221> misc_feature
<223> Primer

<400> 10

gccagaattc aagacaggga gggtatccca cctgaccagc agcgtctgat atttgccggc 60
agacagctgg aggatggccg cactctctca gactacaac 99

<210> 11
<211> 87
<212> DNA
<213> Artificial sequence

<220>
<221> misc_feature
<223> Primer

<400> 11

cactctctca gactacaaca tccagagaga gtccaccctg cacctggtgt tgcgcctccg 60
cggtggtatg gcctcctccg aggacgt 87

<210> 12
<211> 20
<212> DNA
<213> Artificial sequence

<220>
<221> misc_feature
<223> Primer

<400> 12
atggcctcct ccgaggacgt 20

<210> 13
<211> 40
<212> DNA
<213> Artificial sequence

<220>
<221> misc_feature
<223> Primer

<400> 13
ccgccactcc accggcgccg gatctgacta caaggacgat 40

<210> 14
<211> 35
<212> DNA
<213> Artificial sequence

<220>
<221> misc_feature
<223> Primer

<400> 14
acaaggacga tgacgataag tagtaactcg agtca 35

<210> 15
<211> 22
<212> DNA
<213> Artificial sequence

<220>
<221> misc_feature
<223> Primer

<400> 15
cctggagacg ccatccacgc tg 22

<210> 16
<211> 49
<212> DNA
<213> Artificial sequence

<220>
<221> misc_feature
<223> Primer

<400> 16
taatacgact cactataggg agctcctcgc ccttgctcac cattaagct 49

<210> 17
<211> 228
<212> DNA
<213> Artificial sequence

<220>
<223> oligonucleotide

<400> 17

```
atgcagatct ttgtgaggac cctcactggc agaaccatca cccttgaggt cgagcccagt       60
gacaccattg agaatgtcag agccagaatt caagacaggg agggtatccc acctgaccag      120
cagcgtctga tatttgccgg cagacagctg gaggatggcc gcactctctc agactacaac      180

atccagagag agtccaccct gcacctggtg ttgcgcctcc gcggtggt                   228
```

<210> 18
<211> 76
<212> PRT
<213> Artificial sequence

<220>
<223> polypeptide

<400> 18

```
Met Gln Ile Phe Val Arg Thr Leu Thr Gly Arg Thr Ile Thr Leu Glu
1               5                   10                  15
Val Glu Pro Ser Asp Thr Ile Glu Asn Val Arg Ala Arg Ile Gln Asp
            20                  25                  30
Arg Glu Gly Ile Pro Pro Asp Gln Gln Arg Leu Ile Phe Ala Gly Arg
        35                  40                  45
Gln Leu Glu Asp Gly Arg Thr Leu Ser Asp Tyr Asn Ile Gln Arg Glu
    50                  55                  60
Ser Thr Leu His Leu Val Leu Arg Leu Arg Gly Gly
65                  70                  75
```